# EUROPÄISCHE PATENTSCHRIFT

(11) **EP 3 600 156 B1**
(45) Veröffentlichungstag und Bekanntmachung des Hinweises auf die Patenterteilung: **13.11.2024**
(21) Anmeldenummer: 18717859.5
(22) Anmeldetag: 28.03.2018
(51) Int. Cl.: A61F 2/24

(54) **HERZKLAPPEN-IMPLANTAT UND HERZKLAPPEN-IMPLANTAT-SYSTEM**
HEART VALVE IMPLANT AND HEART VALVE IMPLANT SYSTEM
IMPLANT DE VALVULE CARDIAQUE ET SYSTÈME D'IMPLANT DE VALVULE CARDIAQUE

(30) Priorität: 28.03.2017 DE 102017002974
(43) Veröffentlichungstag der Anmeldung: 05.02.2020
(73) Patentinhaber: Immanuel Albertinen Diakonie gGmbH, 22457 Hamburg (DE)
(72) Erfinder: ALBES, Johannes, Prof. Dr. med., 13465 Berlin (DE)
(74) Vertreter: Kilger, Ute
(86) Internationale Anmeldenummer: PCT/EP2018/058028
(87) Internationale Veröffentlichungsnummer: WO 2018/178210

(56) Entgegenhaltungen:
- WO-A1-2015/020971
- US-A1- 2014 067 048
- US-A1- 2015 073 547

## Beschreibung

Die Erfindung betrifft ein Herzklappen-Implantat und ein Herzklappen-Implantat, insbesondere für die minimal-invasive Herzchirurgie.

### Hintergrund

Im herzchirurgischen Bereich werden Instrumente, Vorrichtungen oder Verfahren eingesetzt, um das Innere von lebenden Organismen, zum Beispiel das Innere des Herzens, zu untersuchen und/oder für operative Eingriffe zu nutzen, beispielweise die minimal-invasive Reparatur von Herzklappen, wobei chirurgische Instrumente verwendet werden, die es mit Hilfe eines Zugangs in das Herz erlauben, verschiedene Reparaturen und den Einsatz von Implantaten am schlagenden Herzen durchzuführen.

Verschiedenartige konventionelle und minimal-invasive chirurgische Verfahren kommen derzeit bei einem Herzklappeneingriff zur Anwendung. Herzklappeneingriffe sind kathedergestützte oder operative Eingriffe an den Herzklappen bzw. Herzklappensegeln, mit dem Ziel, die Funktionsfähigkeit einer Herzklappe wieder herzustellen. Zur Herstellung der Funktionsfähigkeit stehen verschiedene technische Verfahren und chirurgische Instrumente zur Verfügung. Solche Techniken umfassen die Reparatur und den Ersatz von Herzklappen. Um eine Reparatur am Herzen vornehmen zu können, gibt es verschiedene Zugangswege. Ein operativer Zugangsweg zum Herz erfolgt zum Beispiel über die Thorakotomie in Form einer medianen Sternotomie, die den Zugang in die Patientenbrusthöhle ermöglicht. Hierzu muss das Sternum der Länge nach aufgeschnitten bzw. aufgesägt werden und mit einem Rippenspreizer werden die beiden Hälften des Brustkorbes auseinander gedehnt. Dem Operationsteam eröffnet sich jetzt eine freie Sicht auf das Herz und die thorakalen Gefäßsysteme. Aufgrund der guten Visualisierung und Größe des Operationsfeldes, kann eine Vielzahl von chirurgischen Instrumenten eingesetzt werden. Eine solche Öffnung des Brustkorbes verursacht aber bei einem Patienten ein hohes Maß an Traumatisierung, längere Liegezeiten im Krankenhaus und einen längeren Heilungsprozess. Dieses bekannte Zugangsverfahren und die hierzu verwendeten chirurgischen Instrumente werden hier nur aufgezeigt, um den Stand der Technik zu dokumentieren, sie sollen aber nicht weiter betrachtet werden.

Bei manchen Herzerkrankungen bzw. bei Herzinsuffizienzen wird der Eingriff am Herzen mit Hilfe von Kathetern durchgeführt. Manche Herzklappenfehler lassen sich durch moderne Katheter-Verfahren auf schonende Weise beheben und können eine größere Operation mitunter vermeiden. Insbesondere werden heutzutage Defekte an den Herzventilen der linken Herzhälfte, also an der Aorten- und Mitralklappe, mit Hilfe eines Katheters behandelt. Wie bei anderen Katheter-Interventionen auch, wird ein Kunststoffkatheter über ein Blutgefäß in der Leiste oder im Arm bis zum Herzen vorgeschoben. Auch dieses Zugangsverfahren (Transkatheter-Technologie) zum Herzen soll hier nicht näher betrachtet werden.

Für eine Vielzahl von Herzerkrankungen bzw. Herzinsuffizienzen erfolgt der Zugang zum Herzen mit Hilfe der minimal-invasiven Methode, insbesondere bei der Mitralklappenchirurgie. Bei der Mitralklappenchirurgie war bisher das Öffnen des Brustkorbes eines Patienten und der Einsatz einer Herz-Lungen-Maschine noch notwendig.

Aus dem Stand der Technik ist bekannt, dass solche Operationen bei Herzklappeneingriffen auch am schlagenden Herzen, siehe die Offenbarung in der WO 2006/078694 A2, vorgenommen werden können. Rekonstruktionen und Ersatz sind durch Einsatz der minimal-invasiven Chirurgie, wie beim offenen Thorax-Verfahren, also möglich.

Zu unterscheiden ist zwischen einer Aortenklappen-Rekonstruktion und einer Mitralklappen-Rekonstruktion. Bei der Mitralklappen-Rekonstruktion handelt es sich um eine Wiederherstellung der Ventilfunktion mit Erhalt der Mitralklappe (Bikuspidalklappe). Zur erfolgreichen Reparatur der Ventilfunktion einer Mitralklappe im Inneren eines menschlichen Herzens, sind daher die verschiedenen Komponenten der Mitralklappe zu untersuchen und deren mögliche Fehler zu verifizieren. Die Untersuchung erfolgt u.a. mit Hilfe der Diagnostik vor der Operation, z. B. mit einem Herzkatheter und die Echokardiographie.

Die Mitralklappe (Mitral Valve) besteht aus vier funktionellen Komponenten, den beiden Segeln (Mitral Valve Leaflets) bestehend aus einem vorderen Segel (cupis anterior) und einem hinteren Segel (cupis pasterior), der Aufhängung der Segel im Mitralklappenring (Mitral Valve Annulus), den Sehnenfäden (Chordae tendineae), mit welchen die Segel an den Papillarmuskeln (Papillary Muscles) beweglich befestigt sind und den Papillarmuskeln selbst, die im Myokard enden. Zur Reparatur jeder einzelnen Komponente steht ein unterschiedliches chirurgisches Instrument und/oder ein Implantat zur Verfügung.

Zur Mitralklappen-Rekonstruktion gehört auch die Reparatur der Sehnenfäden, z. B. durch Implantation von künstlichen Fäden als Ersatz. Aus dem Stand der Technik, der US 8,758,393 B2 und der US 9,192,374 B2, ist eine Vorrichtung zur minimal-invasiven Reparatur von Sehnenfäden eines (prolapsed) Mitralklappensegels bekannt. Als Sehnenfadenruptur bezeichnet wird man die Zerreißung einer oder mehrerer an den Segeln der Mitralklappe inserierenden Chordae tendineae. Hierbei wird ein gerissener Sehnenfaden (severed chordae), der eine Klappenregurgitation verursacht, durch einen künstlichen Faden (artificial chodae) ersetzt, wobei der künstliche Faden einerseits am Segel der Mitralklappe des linken Vorhofes und andererseits am Epikard der Spitze (des Apex) der linken Herzkammer (left ventricel), befestigt wird, um das Zurückschlagen der Segelklappe (valve leaflets) in den Vorhof (left atrium) während der Systole zu verhindern. Der Zugang des Instrumentes zum Einsetzen eines Implantates aus künstlichen Sehnenfäden (artificial chordae) erfolgt über eine Inzision (lateral LV incision by the true apex) durch das Myokard an der Spitze (apex) des Herzens in den linken Ventrikel. Um an die Stelle eines gerissenen Sehnenfadens (severed chordae) im Herzens mit der minimalen-invasiven Mitralklappen-Chirurgie zu gelangen, ist es notwendig, eine linke seitliche Brustkorböffnung (left anterolaterale minithoracotomy) vorzunehmen. Durch die Öffnung im Apex wird das Instrument in den linken Ventrikel eingeführt und damit das durch Insuffizienz geschädigte Klappensegel erfasst. Das Instrument führt einen zweifachen künstlichen Sehnenfaden durch das Klappensegel und befestigt diesen mit Hilfe einer Schlaufe, wodurch das Klappensegel erfasst wird. Die beiden Enden des Sehnenfadens werden außerhalb des Epikards am Apex verknotet, nachdem die notwendige Länge des Sehnenfadens über verschiedene Messverfahren, z. B. die Echokardiografie gemäß dem TEE-Verfahren, ermittelt wurde. Vorher wurde noch die Öffnung an der Herzspitze vernäht. Dieses Instrument ist aber nicht für den Einsatz eines gerissenen Sehnenfadens (severed chordae) im linken Ventrikel des Herzens geeignet, wenn der Zugang über den linken Vorhof erfolgt.

Ein weiteres Herzklappen-Reparatur-System am schlagenden Herzen für den Einsatz der minimal-invasiven Chirurgie, ist der US 9,044,221 B2 zu entnehmen. Es wird ein chirurgisches Verfahren unter Verwendung eines austauschbaren Reparatursystems beschrieben. Das Reparatursystem besteht aus verschiedenen Komponenten, die zu einer Vorrichtung zusammengestellt werden. Für die Anwendung des Verfahrens ist ein Zugang zwischen den Rippen im linken Thoraxbereich erforderlich, um die Spitze der Herzwand öffnen zu können und mit einem Zugang zu versehen. Der Zugang ist in der Lage, ähnlich wie bei einem Trokar, verschiedene Komponenten der Vorrichtung aufzunehmen. Die zusammengestellte und verriegelte Anordnung der Vorrichtung wird dann als Einheit durch den Zugang in die linke Herzkammer vorgeschoben. Die Überwachung des Vorschiebens der Vorrichtung erfolgt durch bildgebende Verfahren. Mit der Vorrichtung kann nach Ergreifen des Gewebes ein künstlicher Faden, mit Hilfe einer Nähkassette, einerseits am Herzklappensegel mit einem Knoten (girth hitch knote) befestigt und andererseits an einem Papillarmuskel angenäht werden, um eine Klappen-Regurgitation zu reduzieren. Auch die Verwendung eines Knotenschiebers und die Verknotung der Fäden auf dem Epikard auf der Außenseite des Herzens im Bereich des Apex sind möglich. Auch dieses Instrument und Implantat ist nicht für den Einsatz eines gerissenen Sehnenfadens (severed chordae) im linken Ventrikel des Herzens geeignet, wenn der Zugang über den rechten Thoraxbereich in den linken Vorhof erfolgt.

Die Reparatur und/oder Korrektur der dysfunktionellen Herzklappen kann auch durch den Einsatz eines Herzklappenimplantats, wie in der US 8,480,730 B2, der US 8,888,844 B2, der US 8,894,705 B2 und der US 9,232,999 B2 offenbart, erfolgen. Hier geht es nicht um die Anwendung erfinderischer chirurgischer Instrumente bzw. Vorrichtungen, mit deren Hilfe Mitralklappeninsuffizienzen beseitigt werden können, sondern um einen Mitral-Spacer. Der Mitral-Spacer ist ein Ventilimplantat, das in einer sich öffnenden und schließenden Öffnung einer Mitralklappe eingesetzt werden kann, um bei einer Kontraktion des linken Ventrikels einen Rückfluss von Blut aus dem Ventrikel in das linke Atrium zu verhindern. Das Ventilimplantat besteht aus einer Welle, die sich entlang einer Längsachse des Herzimplantats erstreckt und die am oberen Ende einen Abstandshalter aufweist, der aus einer Vielzahl von Segmenten gebildet ist. Die Segmente können eine unterschiedliche Größe und Form aufweisen. Die Außenflächen der Segmente dienen zum Anliegen der Klappensegeln beim Schließen der Mitralklappe. Die Welle weist am unteren Ende einen Ankerabschnitt auf. Der Ankerabschnitt besteht aus einer Wendelschraube (helical tissue anchor), die durch Drehen um ihre Achse in das Muskelgewebe des Herzens in Eingriff gebracht wird. Wie und mit welchen Mitteln eine Wendelschraube im Muskelgewebe befestigt wird, ist nicht offenbart. Das Einsetzen eines solchen Ventilimplantats erfolgt über den Zugang der medianen Längssternotomie, die es ermöglicht, das Herz in die entsprechende Position zu bringen oder über den Zugang der rechten Thorakotomie. Beide Verfahren ermöglichen den Zugriff auf das linke Atrium des Herzens mit Sicht auf die Mitralklappe. Ein den Chirurgen bekannter Katheter wird benutzt, um das Ventilimplantat ins linke Atrium einzuführen, dort zu befestigen und den Ventilkörper zwischen den beiden Mitralklappensegeln zu platzieren. Eine Befestigung der Klappensegel am Ventilkörper bzw. Abstandshalter erfolgt nicht.

Aus der US 8,216,302 B2 ist ein Zuführkatheter bekannt, der perkutan ins Herz eingeführt und durch den das Mitralklappen-Implantat vorgeschoben wird. Die Befestigung des Mitralklappen-Implantats im linken Ventrikel erfolgt durch einen Verankerungsmechanismus, der eine Wendelschraube enthält. Die Wendelschraube wird am nativen Herzgewebe, der Muskelwand des linken Ventrikels nahe der Spitze des Herzens, eingebracht. Das Einbringen der Wendelschraube erfolgt durch Drehen des Implantats, wodurch die Wendelschraube in das Muskelgewebe eindringt. Das Einbringen der Wendelschraube kann auch gemäß der Beschreibung der Fig. 7 und Fig. 8 erfolgen. Ein Verriegelungsmechanismus, bestehend aus zwei Verriegelungsstiften, die sich in gekoppelter Position in einer Hülse befinden, können mit Hilfe eines Zuführdrahtes, der durch die Zentriersätze in der Hülse geführt wird, gedreht und verschoben werden. Der Verriegelungsmechanismus wirkt auf einen Verankerungsdraht und einen Stoppmechanismus ein, um die in das Gewebe einzubringende Wendelschraube zu steuern. Um den komplizierten Mechanismus zu vermeiden, ist es notwendig, ein neues Einschraubsystem zu entwickeln.

Das Einbringen einer Wendelschraube in ein Gewebe mit Hilfe einer Vorrichtung, ist auch aus der US 2007/0150000 A1 bekannt. Mit der Vorrichtung werden zwei beabstandete Gewebelappen in einem Herzen mit einer Wendelschraube miteinander verbunden. Hierzu wird durch einen transvenös in das Herz einführbaren Zuführkatheter eine verschiebbare Vorrichtung (Schraubkatheter) an die beabstandeten Gewebelappen herangeführt und durch die eindrehbare Wendelschraube zusammengeführt. Die jetzt anliegenden Gewebelappen werden durch Anlegen einer Hochfrequenzspannung miteinander verklebt.

In der Herz-und Thoraxchirurgie wird meistens eine offene Operation durchgeführt, bei der, durch die Eröffnung des Thorax, ein Zugang zum Herzen geschaffen wird. Der Zugang erfolgt in der Regel mittels einer medianen Sternotomie, wobei zum Öffnen des Brustkorbes ein, in etwa 25 cm langer Längsschnitt durch das Brustbein erzeugt wird. Bei der Thorakotomie erfolgt die chirurgische Öffnung des Thorax durch einen Interkostalschnitt, d. h. durch einen kleinen Schnitt in den Rippenzwischenraum. Die durch die Sternotomie oder Thorakotomie erzeugte Öffnung wird von einem Rippenspreizer, der zum Aufdehnen und Offenhalten des Brustkorbes eingesetzt wird, freigehalten. Die Öffnung dient den Chirurgen als Zugang für operative Eingriffe. Die Eingriffe an den organischen Körperteilen erfolgen dann mit Hilfe einer Vielzahl unterschiedlicher chirurgischer Instrumente durch die erzeugte Öffnung im Brustkorb. Ist beispielsweise das Herz des Patienten freigelegt, werden direkt an das Herz und die großen Blutgefäße verschiedene Katheder, Kanülen und Klemmen angelegt. Typischerweise wird die Aorta mit einer Gefäßklemme rund um die aufsteigende Aorta okkludiert, um die Koronararterien vom Rest des Arteriensystems zu isolieren, wobei hier unter Okkludieren das Ergreifen, das Zusammendrücken, das Klemmen und Halten eines Gefäßes verstanden wird. Die notwendigerweise eingesetzten chirurgischen Instrumente verkleinern die Körperöffnung und behindern somit die Tätigkeit des Chirurgen in seinem Gesichtsfeld. Außerdem ist aufgrund der Größe der Öffnung, des entstandenen Gewebeschadens und des operativen Traumas, ein schneller Heilungsprozess bei dem Patienten nicht zu erwarten. Die Nachteile einer medianen Sternotomie sind zu vermeiden.

Um die, aus der minimal-invasiven Chirurgie gestellten Anforderungen an die Herzklappen-Implantate und zugehörigen chirurgischen Instrumente zu erfüllen, ist es notwendig, neue Ausführungsformen von Herzklappen-Implantaten und chirurgischen Instrumenten zu entwickeln.

Es besteht Bedarf für ein medizinisches Herzklappen-Implantat mit chirurgischen Instrumenten, für den Einsatz in der minimal-invasiven Chirurgie zu schaffen, welches die vorgenannten Nachteile und Unzulänglichkeiten der bekannten Anordnungen vermeidet, insbesondere ein chirurgisches Herzklappen-Implantat, welches einerseits einfach und kostengünstig in der Herstellung ist, und andererseits es ermöglicht, ein, in ergonomischer- und Handlings-Hinsicht mit einfacher Funktionsgeometrie, ausgestattetes Herzklappen-Implantat für die erhöhten Ansprüche herzustellen. Dieses chirurgische Herzklappen-Implantat soll nicht nur organische Körperteile wieder rekonstruieren, sondern es soll dem Chirurgen auch die Möglichkeit gegeben werden, aufgrund unterschiedlicher Gegebenheiten im Herzen von Patienten, z.B. auf unterschiedliche Längenabstände zwischen dem Myokard und einem Mitralklappensegel eingehen zu können und einen minimierten Rückfluss im Ventil der Mitralklappe einstellen zu können. Der unterschiedlich einstellbare Rückfluss soll diversen medizinischen Einsätzen entsprechen.

Daher besteht Bedarf, eine Operation mit Hilfe der minimal-invasiven Technik (Minithorakotomie) am schlagenden Herzen zu ermöglichen. Natürlich hat der behandelnde Herzchirurg vorab den Patienten geprüft, ob eine Herzklappenreparatur mit einem minimal-invasiven Verfahren durchgeführt werden kann. Anatomische oder technische Voraussetzungen, aber auch die Komplexität des notwendigen Eingriffs begrenzen den Einsatz minimal-invasiver Verfahren erheblich.

Aus dem Stand der Technik sind Herzklappen-Implantate zur minimalinvasiven Reparatur einer Ventilklappe im schlagenden Herzen eines Patienten, wie zuvor aufgezeigt, bekannt. Ein Herzklappen-Implantat, insbesondere für die Mitralklappen-Rekonstruktion, besteht beispielsweise aus einem Verbindungselement, wie Faden, Welle oder Draht, das sich allgemein linear entlang einer Längsachse des Herzklappen-Implantats erstreckt, wobei das Verbindungselement mit einem ersten und einem zweiten Ende, die einander gegenüber liegen angeordnet sind, einem Anker, vorzugsweise ausgebildet als Wendelschraube, die ein proximales und ein distales Ende aufweist, wobei das proximale Ende am ersten Ende des Verbindungselementes angeordnet ist. Am zweiten Ende des Verbindungselementes befindet sich ein Befestigungsmittel. Ein solches Herzklappen-Implantat wird vom linken Thoraxbereich in den linken Ventrikel eingesetzt.

Aus dem Dokument US 2015 / 0073547A1 ist ein Herzimplantat bekannt, welches mit Hilfe eines Gewebeankers am Herzgewebe befestigt wird. Bei dem Gewebeanker ist eine Wendelschraube mit einem Schaft-Bauteil verbunden. Auf diese Weise sind ein Verbindungselement und ein Verankerungselement bereitgestellt. Über das Verbindungselement ist die Wendelschraube mit einem Ventilkörper des Implantats verbunden.

### Zusammenfassung

Es ist Aufgabe der Erfindung, ein Herzklappen-Implantat anzugeben, welches im Rahmen der Anwendung der minimal-invasiven Chirurgie über den rechten Thoraxbereich und den linken Vorhof des Herzens in den linken Ventrikel mit Hilfe bekannter Katheter eingebracht und dort verankert werden kann.

Ein Implantat soll daher nur die Größe annehmen, die an die Operationsstelle durch einen Trokar und/oder Katheter geführt werden kann. Das Implantat soll in einer Ausgestaltung mit einem Befestigungsmittel ausgestaltet sein. Das Befestigungsmittel soll eine Mitralklappe bzw. ein Mitralklappensegel erfassen können. Die Mitralklappe bzw. ein Mitralklappensegel muss mit dem Befestigungsmittel weiterhin beweglich bleiben, aber im Bewegungsspielraum einstellbar und begrenzt. Eine Verbindung des Befestigungsmittels mit dem Myokardgewebe des Herzens kann vorgesehen sein.

Zur Lösung sind ein Herzklappen-Implantat sowie ein Herzklappen-Implantat-System nach den Ansprüchen 1 und 10 geschaffen. Ausgestaltungen sind Gegenstand von Unteransprüchen.

Es kann ein Hybrid-OR-Szenario bei einem anästhesierten Patienten zur Herzklappen-Reparatur angewendet werden. Danach werden bei kollabiertem rechten Lungenflügel mehrere seitliche kleine Zugangsöffnungen im rechten Brustkorb zwischen dem 3'ten oder 4'ten Rippenzwischenraum eingebracht. Dieser Eingriff erfolgt mit der minimal-invasiven Technik (auch Schlüsselloch-Chirurgie genannt) und nimmt beispielsweise Trokare, Wundspreizer, Optiken, einen Vorhofdach-Retraktor u.a. Instrumente, auf. Vorteilhafterweise werden Zugänge, z.B. für eine Aortenklemme und für eine Herz-Lungen-Maschine bei der Anwendung des minimal-invasiven chirurgischen Verfahrens zum Implantieren eines Herzklappen-Implantats nicht mehr benötigt, wodurch eine Verringerung der Invasivität und damit eine Entlastung für den Patienten erreicht werden.

Um mit den chirurgischen Instrumenten und Implantaten in das Herz eindringen zu können und einen Herzklappenvorfall, insbesondere einen Mitralklappenvorfall, zu beheben, ist es notwendig, zur Durchführung einer Mitralklappenrekonstruktion den linken Vorhof mit einem kleinen Schnitt, einer Inzision, zu öffnen und einen Trokar einzusetzen. Der Trokar dient z.B. der Aufnahme und als Zugangsführung für einen oder mehrere Katheter sowie für ein Implantat in den linken Vorhof und anschließend weiter durch die Öffnung, die sich im Ventil einer Mitralklappe bzw. zwischen den Mitralklappensegeln ergibt, um in den linken Ventrikel der Mitralklappe vordringen zu können.

Das Herzklappen-Implantat weist in einer Ausgestaltung ein Mitralklappen-Implantat auf, das auch mit dem Produktnamen "MitraPeg" bezeichnet werden kann. Das "MitraPeg" kann mit drei Elementen gebildet sein. Ein erstes Element ist ein spiralförmig ausgebildetes Verankerungselement, das als Wendelschraube ausgebildet ist. Das zweite Element ist ein Verbindungselement, bestehend aus einem künstlichen Faden oder Draht, der mit einem Klemmmittel in Form eines Gleitrings ausgestattet ist. Der Gleitring stellt die Verbindung zwischen einem Faden und einem Befestigungsmittel her. Das dritte Element bildet die Basis des Mitralklappen-Implantats, es betrifft ein Befestigungsmittel, um die Bewegung einer Mitralklappe einzugrenzen bzw. positionieren zu können. Alle drei Elemente sind nach der Konfektionierung miteinander zu einem Herzklappen-Implantat verbunden.

Das Befestigungsmittel kann einerseits ein Klappensegel einer Mitralklappe erfassen und andererseits eine Verbindung zum künstlichen Faden oder Draht herstellen, an dessen Ende das Verankerungselement angeordnet ist. Das Befestigungsmittel kann wiederum drei Elemente aufweisen. Die drei Elemente können ein Rohrelement, ein Verbindungselement und ein Greifelement umfassen. Das Rohrelement kann einer zylindrische Hülse mit einem, daran angeordneten Verbindungselement aufweisen. Das Verbindungselement kann mit einem drahtartigen Bügel gebildet sein, der mit der zylindrischen Hülse gelenkartig in Verbindung steht. Ein Gelenk ist in der Technik eine drehbewegliche Verbindung zwischen zwei Teilen mit einem Freiheitsgrad. Die Verbindung kann derart gestaltet sein, dass der Bügel sich um 360 Grad um die Hülse schwenken lässt. Der Bügel kann hierzu annähernd u-förmig ausgebildet sein, wobei das eine offene Querende des Bügels mit jeweils einem Zapfen in jeweils eine Öffnung in der Hülsenwand drehbeweglich eingreift und somit schwenkbar an der Außenseite der Hülse anordbar ist. Die beiden Öffnungen in der Hülsenwand verlaufen quer durch die Hülse bzw. senkrecht zur Längsachse der Hülse und sind, in Längsrichtung der Hülse betrachtet, ungefähr mittig angeordnet.

Das andere, von der Hülse entfernte Querende des drahtartigen Bügels, kann frei von Zapfen sein und eine durchgehende Querstange aufweisen, die als Verbindungsstange zwischen den beiden Längsschenkeln angeordnet ist und diese verbindet. Die Verbindungsstange ist Träger eines Greifelementes.

Das Greifelement kann eine Schenkelfeder und zwei Federarme aufweisen. Die Schenkelfeder kann eine Blattfeder aus Edelstahl sein, zum Beispiel aus Federstahl oder Nitinol und kann zwei Ösen oder eine Nute zur Aufnahme der Verbindungsstange des Bügels aufweisen.

Die Verbindungsstange des Bügels bildet gleichzeitig im Scheitel der Schenkelfeder eine Querachse, in welche das von der Hülse entfernte Querende des Bügels, eingreift. Die Verbindung zwischen dem Verbindungselement und der Schenkelfeder kann derart gestaltet sein, dass sich die Schenkelfeder um die Verbindungsstange des Bügels drehen kann. Die Schenkelfeder bildet mit dem Bügel somit eine gelenkartige Verbindung, die senkrecht zur Längsachse der Hülse verläuft. Einerseits kann die Schenkelfeder auf einer festen Kreisbahn in einem gewissen Winkelbereich um die Querachse in der Hülse schwenkbar angeordnet sein, und andererseits kann die Schenkelfeder sich selbst um die eigene Querachse drehen. Aufgrund dieser Konstruktion sind die fest miteinander verbundenen Elemente und Mittel über die gelenkartigen Verbindungen beweglich angeordnet.

Die Schenkelfeder kann wiederum Träger zweier blattförmiger Federarme sein, die fest mit der Schenkelfeder verbunden sind. Die beiden Federarme können durch die Schenkelfeder parallel beabstandet sein und ein Maulteil mit Greifabschnitten aufweisen. Die Aufgabe der Federarme und der Schenkelfeder ist es, das Maulteil des Greifelementes öffnen und schließen zu können. Der Schenkelfeder kommt daher eine besondere Bedeutung zu, weil diese eine Kraft auf die Federarme ausüben muss, um ein Klappensegel zwischen den Federarmen bzw. den Greifarmen des Maulteils dauerhaft einklemmen und festhalten zu können. Die leicht oval gebogenen Federarme haben die Aufgabe, elastische Greifarme zu bilden. Die Federarme sind an einem Ende geöffnet und weisen einen Abstand auf, der durch die Größe der Schenkelfeder vorgegeben wird. Das andere Ende der Federarme bildet die beweglichen Greifarme mit dem geschlossenen Maulteil.

Ein solches Herzklappenimplantat, geeignet für eine Implementierung, wurde nach den vorhergehenden Angaben im Zusammenhang mit einem Mitralklappen-Implantat offenbart. Das Implantat kann auch in anderen Anwendungen eingesetzt werden, beispielsweise als Implantat assoziiert mit einer anderen Herzklappe. Die Aspekte der vorliegenden Offenbarung sind daher nicht auf ein Mitralklappen-Implantat beschränkt, sondern das Implantat kann ausgelegt sein für die Verwendung von verschiedenen Herzklappen-Rekonstruktionen.

Den vorangehenden Erläuterungen entsprechend kann ein solches Herzklappen-Implantat hergestellt und bei operativen Eingriffen am schlagenden Herzen eingesetzt werden. Zuerst galt es die Aufgabe zu lösen, die Baugröße des Herzklappen-Implantats zu minimieren, um einen Zugriff auf das Herz von der rechten Thoraxseite aus zu ermöglichen. Zerlegbarkeit und Minimierung des Herzklappen-Implantats vor dem Einsetzen in einem Herzen sind ermöglicht. Das Herzklappen-Implantat kann konstruktiv mehrteilig ausgeführt sein. Die Elemente des Herzklappen-Implantats werden einzeln in das Herz eingeführt und dort konfektioniert.

Bei den Elementen kann es sich um ein Verankerungselement, ein Verbindungselement mit Klemmmittel und ein Befestigungsmittel handeln. Die drei Elemente zusammen können das Herzklappen-Implantat bilden.

Die Minimierung des Herzklappen-Implantats bedeutet, dass die einzelnen Elemente nur eine maximale Baugröße annehmen können, die noch durch einen, im Trokar geführten chirurgischen Instrument passen. Das Verankerungselement und das Verbindungselement stellen in ihrer Baugröße nicht das Problem dar, sondern die zum Einbringen und Befestigen der Elemente benötigten chirurgischen Instrumente. Daher kann für das Einbringen und Befestigen des, am Verbindungselement angeordneten Verankerungselementes, ein inneres chirurgisches Instrument in Form eines Rohrschiebers II vorgesehen sein. Der innere Rohrschieber II kann mit seinen Abmessungen geeignet sein, durch einen, im Vorhof des Herzens eingesetzten Trokar und durch den, im Trokar eingeführten chirurgischen Instrument in Form eines äußeren Rohrschiebers geführt zu werden. Des Weiteren muss der innere Rohrschieber II durch ein Befestigungsmittel geschoben werden können. Der Rohrschieber wird hier als innerer Rohrschieber II bezeichnet. Er ist derart konstruiert, das er das Verankerungselement mit dem daran angeordneten Verbindungselement durch den linken Vorhof und, den linken Ventrikel bis zum Myokardium im Herz führen und dort befestigen kann. Zum Befestigen des Verankerungselements weist der innere Rohrschieber II am einführenden Ende ein Klemmmittel auf, mit welchem das Verankerungselement in das Myokard eingedreht werden kann. In einfacher Art und Weise kann der innere Rohrschieber II vom Verankerungselement gelöst und aus dem äußeren Rohrschieber entfernt werden.

Dafür, das, am Verankerungselement angeordnete Verbindungselement, mit einer Mitralklappe bzw. einem Mitralklappensegel zu verbinden, wurde ein Befestigungsmittel, wie zuvor beschrieben und in den Figuren aufgezeigt, entwickelt. Das Befestigungsmittel kann eine Baugröße von nur wenigen Millimetern aufweisen, derart konzipiert, dass es, mit Hilfe eines chirurgischen Instruments, in Form eines äußeren Rohrschiebers, durch einen Trokar geführt werden kann. Gemäß eines weiteren Aspekts weist das Befestigungsmittel ein Greifmittel zum Erfassen und Klemmen eines Mitralklappensegels auf. Des Weiteren kann das Befestigungsmittel mit dem Verbindungselement verbunden werden, wodurch eine definierte beabstandete Verbindung zwischen dem Mitralklappensegel und dem Myokard hergestellt werden kann. Die beabstandete Verbindung ist in der Länge fest begrenzt, d.h., ein Mitralklappensegel kann sich nur im Ventrikel bewegen, aber nicht in den Vorhof zurück schwingen. Bei der Verkürzung der Länge ist die Verbindung aber beweglich, d.h., wenn die Mitralklappe im Ventrikel in Richtung Myokard schwingt, gibt das Verbindungselement nach. Eine in der Länge fest begrenzte, aber bewegliche Verbindung zwischen einem Mitralklappensegel und dem Myokard erfolgt durch das Einbringen eines Klemmmittels in das Befestigungsmittel, mit welchem das Verbindungselement im Befestigungsmittel geklemmt wird. Das Klemmmittel besteht aus einem Gleitring. Für das Einbringen des Gleitrings in das Befestigungsmittel steht ein weiterers chirurg. Instrument in Form eines weiteren Rohrschiebers III zur Verfügung. Auch für das Einbringen des Befestigungsmittels in das Herz wird ein spezielles chirurg. Instrument in Form eines äußerer Rohrschieber, wie zuvor aufgezeigt, benötigt. Der äußere Rohrschieber ist durch einen Trokar einführbar. Des Weiteren ist der äußere Rohrschieber in der Lage, die anderen inneren Rohrschieber I, II, III aufnehmen zu können. Beispielsweise wird mit einem inneren Rohrschieber I, der durch den äußeren Rohrschieber geführt wird, das Greifelement des Befestigungsmittels zum Öffnen und Schließen eines Maulteils, erfasst. Währenddessen hält der äußere Rohrschieber das Befestigungsmittel fest.

Die Aufgabe, die minimal-invasive Chirurgie zum Einsetzen eines Herzklappen-Implantats am schlagenden Herzen von der rechten Thoraxseite aus durchzuführen, wird mit dem vorgenannten Ausführungen gelöst.

Um ein Herzklappen-Implantat, insbesondere ein Mitralklappen-Implantat, herzustellen und bei operativen Eingriffen am menschlichen oder tierischen Körper durch Einsatz der minimal-invasiven Chirurgie zu verwenden, kann vorgesehen sein, die zum Einsetzen eines Herzklappen-Implantats benötigten chirurgischen Instrumente derart zu gestalten, dass dem Chirurgen ergonomisch gestaltete Zuführ- und Entnahmevorrichtungen, mit verschiedenen Rohrschiebern, die das Handling erleichtern, zur Verfügung stehen.

Um ein solches Herzklappen-Implantat im Herzen implantieren zu können, steht ein System mit Vorrichtungen und Herzklappen-Implantaten, gemäß Anspruch 10, zur Verfügung, mit welchem eine Mitralklappen-Rekonstruktion ermöglicht wird.

Ein Herzklappen-Implantat-System zur minimal-invasiven Reparatur einer Ventilklappe im schlagenden Herzen eines Patienten, kann Folgendes aufweisen: einem äußeren Rohrschieber mit Lumen zum Führen und Halten eines Befestigungsmittels und aus einem ersten inneren Rohrschieber I mit Lumen zum Öffnen und Schließen eines Greifelementes. Des Weiteren kann ein zweiter innerer Rohrschieber II mit Lumen zum Führen und Eindrehen eines Verankerungselements, sowie ein dritter innerer Rohrschieber III zum Einführen und Positionieren eines Klemmmittels vorgesehen sein. Mit diesen chirurgischen Instrumenten wird ein Herzklappen-Implantat konfektioniert. Das Herzklappen-Implantat kann ein Verbindungselement wie Faden oder Draht aufweisen, der sich allgemein linear entlang einer Längsachse des Herzklappen-Implantats erstreckt, wobei das Verbindungselement mit einem ersten und einem zweiten Ende, im allgemeinen einander gegenüberliegend, ausgestattet ist. Es kann ein Verankerungselement vorgesehen sein, welches als Wendelschraube mit einem proximalen und einem distalen Ende ausgebildet sein kann, wobei das proximale Ende an dem ersten Ende des Verbindungselementes angeordnet ist und am zweiten Ende des Verbindungselementes ein Befestigungsmittel angeordnet ist.

Des Weiteren kann das Herzklappen-Implantat-System ein Befestigungsmittel, ausgebildet als Rohrelement in Form einer zylindrischen Hülse, und einem Verbindungselement und einem Greifelement aufweisen, wobei das Verbindungselement einen Bügel aufweisen kann, der ein freies Ende aufweist, welches schwenkbar in dem Rohrelement angeordnet ist. Am anderen, in Längsrichtung der Längsachse gegenüber liegenden Ende des schwenkbaren Verbindungselements, kann ein Greifelement schwenkbar angeordnet sein, wobei das Greifelement aus einer Schenkelfeder bestehen kann, an der, fest mit dieser verbunden, zwei durch die Schenkelfeder parallel beabstandete Federarme angeordnet sind, um wenigstens teilweise einen Rückfluss von Blut durch das, sich in einer geschlossenen Position befindliche Ventil der Herzklappe, zu minimieren.

Das Greifelement des Herzklappen-Implantat-Systems weist ein durch die Federarme gebildetes Maulteil an der Befestigungsseite des Greifelementes und auf der abgewandten Seite der geöffneten Schenkelfeder sowie auf der Längsachse des Rohrelementes liegend, aufweisen, wobei das Maulteil über mindestens einen, im Maulteil angeordneten Abstandshalter verfügt, der einen vorbestimmten und genau definierten Spalt zwischen den Greifbacken des Maulteils erzeugt, wodurch die, mit einer Verzahnung ausgestatteten Greifbacken nicht unmittelbar aufeinander zu liegen kommen, sondern das Gewebe atraumatisch klemmen.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Schnittansicht einer linken Herzkammer mit linkem Vorhof und mit eingesetztem Mitralklappen-Implantat im linken Ventrikel;
- Fig. 2a: eine schematische Darstellung der drei Elemente des Befestigungsmittels mit geschlossenen Greifarmen in Seitenansicht und ebenfalls in schematischer Ansicht;
- Fig. 2b: ein Befestigungsmittels mit geöffneten Greifarmen;
- Fig. 2c: in schematischer Darstellung das Maulteil eines Greifelementes;
- Fig. 3a: in perspektivischer Ansicht eine Ausführungsform einer Schenkelfeder ohne Federarme gemäß der Fig. 2a, 2b;
- Fig. 3b: in perspektivischer Ansicht eine alternative Ausführungsform einer Schenkelfeder ohne Federarme gemäß der Fig. 2a, 2b;
- Fig. 3c: in perspektivischer Ansicht einer weiteren Ausführungsform einer Schenkelfeder ohne Federarme gemäß der Fig. 2a, 2b;
- Fig. 4a: in schematischer Darstellung ein Befestigungsmittel mit eingeklemmtem Mitralklappensegel;
- Fig. 4b: in schematischer Darstellung ein Befestigungsmittel mit eingeklemmtem Mitralklappensegel beim Schwenkvorgang des Greifelements, um das Rohrelement;
- Fig. 4c: in schematischer Darstellung ein chirurgisches Instrument zur Führung und Befestigung, des am Verbindungselement angeordnetem Verankerungselement;
- Fig. 5: in schematischer Darstellung ein im linken Ventrikel verankertes HerzklappenImplantat; und
- Fig. 6: in schematischer Darstellung die Herstellung einer Verbindung zwischen einem Befestigungsmittel und einem Verankerungselement mit Unterstützung eines Klemmmittels.

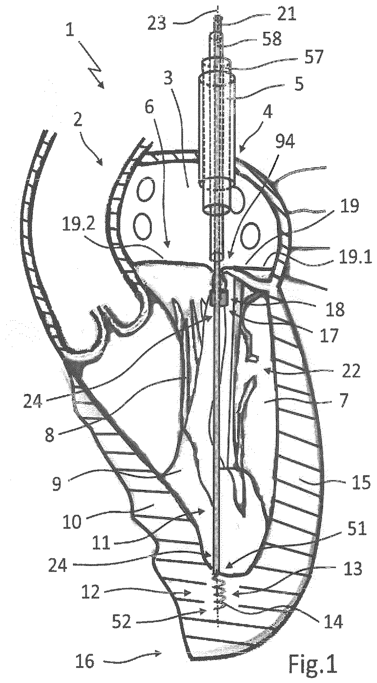

Das in der Fig. 1 in schematischer und prinzipieller Darstellung aufgezeigte Herz 1 liegt, um seine Längsachse gedreht, im linken Brustraum, sodass die rechte Herzhälfte mehr an der vorderen Brustwand anliegt, während die linke Herzhälfte eher nach hinten zeigt. Ausgehend vom bekannten Stand der Technik ist es die Aufgabe der Erfindung, ein Herzklappen-Implantat 11 zu entwickeln, welches bei der Anwendung der minimal-invasiven Chirurgie am schlagenden Herzen 1 eines Patienten über den rechten Thoraxbereich und den linken Vorhof 3 des Herzens 1 und von dort in den linken Ventrikel 7 mit Hilfe bekannter chirurgischer Instrumente und einem Trokar 5 eingebracht und dort verankert werden kann.

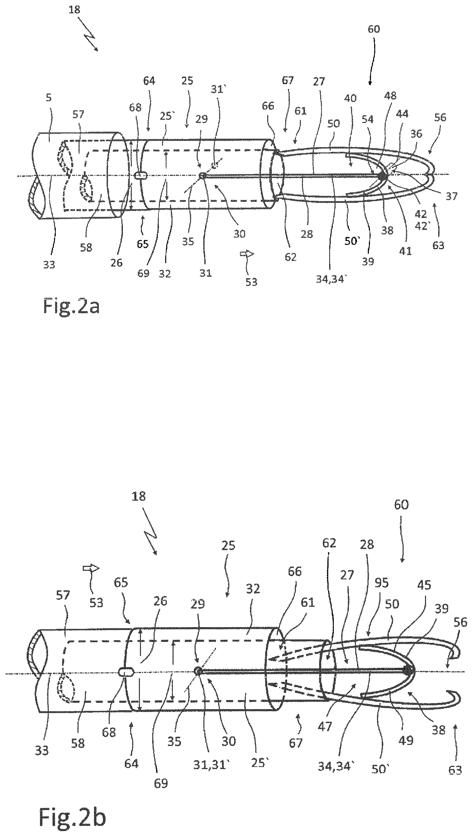

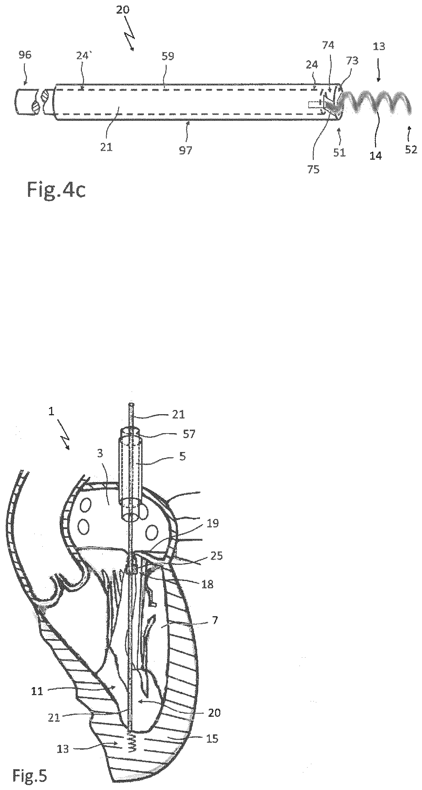

Aufgezeigt ist daher die linke Herzkammer 2 mit linkem Vorhof 3 und einem Zugang 4 in den linken Vorhof 3 zur Mitralklappe 6 sowie dem linken Ventrikel 7. Der Zugang 4 erfolgt über den angedeutetem Trokar 5, einem äußeren Rohrschieber 57 und einem inneren Rohrschieber I 58. Der äußere Rohrschieber 57 wird durch einen Trokar 5 und der innere Rohrschieber I 58 durch den äußeren Rohrschieber 57 geführt. Der innere Rohrschieber I 58 wird im Verlaufe der Operation durch einen anderen Rohrschieber II 59, siehe Fig. 4c, getauscht. Der linke Ventrikel 7 wird in eine Ein- und Ausstrombahn gegliedert. Er ist vom Vorhof 3 durch die Mitralklappe 6 getrennt. Die Mitralklappe 6 ist durch Sehnenfäden (Chordae tendineae) 8 mit den Papillarmuskeln 9 verbunden, die an der Ventrikelwand 10 entspringen und dafür sorgen, dass die Mitralklappe 6 bei ihrem Ventilschluss und während der Anspannungsphase (Systole) der linken Kammer 7 nicht zu heftig in den linken Vorhof 3 zurückschlägt. Im linken Ventrikel 7 ist das eingesetzte Mitralklappen-Implantat 11 zu ersehen. Das Mitralklappen-Implantat 11 weist am distalen Ende 12 ein Verankerungselement 13 auf, wobei das Verankerungselement 13 aus einer korkenzieherartigen Wendelschraube 14 besteht. Der Einsatz andere Ankermittel aus dem bekannten Stand der Technik ist denkbar. Die eingedrehte Wendelschraube 14 befindet sich im Herzmuskelgewebe 15 im Bereich des Apex, dem sogenannten spitzen Herzbereich 16. Des Weiteren weist das Mitralklappen-Implantat 11 am proximalen Ende 17 ein Befestigungsmittel 18 auf, das an einem Mitralklappensegel 19 befestigt ist. Eine Mitralklappe 6 besteht aus zwei Segeln 19.1, 19.2, dem vorderen Segel (Cuspis anterior) 19.1 und dem hinteren Segel Cuspis posterior) 19.2. Gemäß der Fig. 1, ist das Mitralklappen-Implantat 11 beispielhaft am vorderen beschädigten Segel 19.1 angebracht. Zwischen dem Verankerungselement 13 und dem Befestigungsmittel 18 ist ein Verbindungselement 20 angeordnet. Das Verbindungselement 20 besteht aus einem künstlichen Faden 21, der sich allgemein linear entlang einer Längsachse 23 des Herzklappen-Implantats 11 erstreckt und z.B. das Fehlen der Funktion eines oder mehrerer gerissenen Sehnenfäden 22 ersetzt, wobei das Verbindungselement 20 mit einem ersten 24 und einem zweiten Ende 24' im allgemeinen einander gegenüberliegend angeordnet ist und ein Befestigungsmittel 18 mit dem Verankerungselement 13 verbindet. Das Befestigungsmittel 18 wird in Fig. 2a und Fig. 2b näher beschrieben. Analoge Bezugszeichen aus der Fig. 1 werden in den Fig. 2a und 2b übernommen.

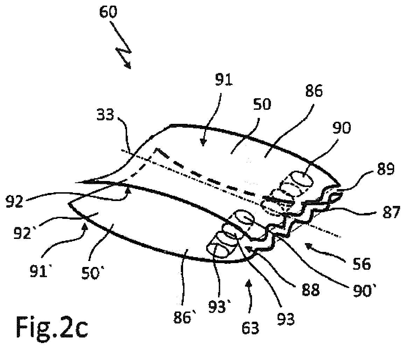

Die Fig. 2a und Fig.2b zeigen, in schematischer Darstellung und Seitenansicht das Befestigungsmittel 18. Gemäß Fig. 2a ist ein geschlossenes Maulteil 56 vorgesehen, und nach Fig. 2b ist ein geöffnetes Maulteil 56 am Greifelement 60 der Federarme 50, 50' vorgesehen. Das Maulteil 56 bildet die aktive Seite des Herzklappen-Implantats 11 bzw. die Befestigungsseite 63 des Herzklappen-Implantats 11 an der Mitralklappe 6 und wird in der Fig. 2c näher beschrieben. Die der Befestigungsseite 63 gegenüberliegende Flanschseite 64 am zylindrischen Rohrelement 25 stellt die passive Seite dar, sie dient zum Handling bzw. dem Einbringen des Befestigungsmittels 18 in den linken Vorhof 3 und im Anschluss daran, nach dem Erfassen und Klemmen eines Mitralklappensegels 19, dem Einbringen in den linken Ventrikel 7.

Vor der Einführung des Befestigungsmittels 18 durch einen Trokar 5 in den Vorhof 3, wird das zylindrische Rohrelement 25 des Befestigungsmittels 18 an einem äußeren Rohrschieber 57 mit einer bekannten Befestigungsart lösbar verbunden. Das lösbare Verbindungselement 68 (nicht näher dargestellt) zwischen dem Rohrelement 25 befindet sich an der Flanschseite 64 des Rohrelements 25 und der Andockseite 65 des äußeren Rohrschiebers 57. Der äußere Rohrschieber 57 ist ein chirurgisches Instrument (hier nicht näher dargestellt), welches vom Operateur außerhalb des Brustkorbes des Patienten bedient wird. Der Außendurchmesser des äußeren Rohrschiebers 57 ist dem Außendurchmesser 26 des zylindrischen Rohrelements 25 weitestgehend angepasst.

Im nächsten Schritt wird das eine freie Ende 61 des Greifelements 60 des Befestigungsmittels 18 mit einem inneren Rohrschieber I 58, der durch den äußeren Rohrschieber 57 und das zylindrische Rohrelement 25 geführt wird, erfasst. Der Außendurchmesser des inneren Rohrschiebers I 58 ist dem Innendurchmesser 66 des dünnwandigen Rohrelements 25 weitestgehend angepasst. Die Erfassung des Greifelements 60 erfolgt dadurch, dass die Öffnung 62 des inneren Rohrschiebers I 58 die Federarme 50, 50' am freien Ende 61 des Greifelements 60 aufnimmt. Hier verhält es sich so, dass der Innendurchmesser 69 der Öffnung 62 des inneren Rohrschiebers I 58 etwas kleiner ist, als der Außenumfang des Greifelementes 60 und somit auch der Federarme 50, 50'. Die Aufnahme der Federarme 50, 50' des Greifelements 60 in die Öffnung 62 des inneren Rohrschiebers I 58, erfolgt durch das Verschieben des inneren Rohrschiebers I 58 über die Federarme 50, 50' des Greifelements 60. Die Verschiebung erfolgt, gemäß der Fig. 2a, nur soweit, bis die Federarme 50, 50' in der Öffnung 62 etwas geführt werden. Hierzu werden, beim Verschieben des inneren Rohrschiebers I 58, die parallel beabstandeten Federarme 50, 50' etwas gegeneinander zusammengedrückt, aber nur so weit, dass einerseits die Federarme 50, 50' gerade in der Öffnung 62 des inneren Rohrschiebers I 58 aufgenommen werden können und andererseits sich das Maulteil 56 des Greifelements 60 noch nicht öffnet. Das Zusammendrücken der Federarme 50, 50' wird ermöglicht, weil die Federarme 50, 50' auf einer Schenkelfeder 38 angeordnet sind. Die Schenkelfeder 38 lässt sich bei Druckausübung auf die Federarme 50, 50' zusammendrücken. Diese Druckausübung auf die Federarme 50, 50'erfolgt mit Hilfe des inneren Rohrschiebers I 58. Nach der Aufnahme der beiden Federarme 50, 50' in die Öffnung 62 des inneren Rohrschiebers I 58, ist das zylindrische Rohrelement 25 auf der Befestigungsseite 63 des Befestigungsmittel 18 annähernd bündig mit dem inneren Rohrschieber I 58.

Die Konfektionierung des Befestigungsmittels 18 an den beiden Rohrschiebern 57, 58 kann auch in umgekehrter Reihenfolge erfolgen, in dem die Federarme 50, 50' zuerst mit dem inneren Rohrschieber I 58 erfasst werden. Die Erfassung der Federarme 50, 50' erfolgt, indem der innere Rohrschieber I 58 durch den Innendurchmesser 66 des zylindrischen Rohrelements 25 von der Flanschseite 64 in Längsrichtung 53 bis zur Greifarmseite 67 geschoben wird. Im nächsten Schritt wird der äußere Rohrschieber 57 am zylindrischen Rohrelement 25 befestigt, indem dieser mit seiner Andockseite 65 voraus, über das innere Rohrelement I 58 bis zur Flanschseite 64 des zylindrischen Rohrelements 25 geschoben und mit dem Verbindungselement 68 verbunden wird. Ist das Befestigungsmittel 18 von beiden Rohrschiebern 57, 58 erfasst, kann es durch den Trokar 5 in den linken Vorhof 3 eingeführt werden. Im linken Vorhof 3 wird dann das Befestigungsmittel 18 zum Ergreifen eines Mitralklappensegels 19 mit Hilfe der beiden Rohrschieber 57, 58 vorbereitet.

Ist das Befestigungsmittel 18 von beiden Rohrschiebern 57, 58 in den linken Vorhof 3 eingeführt, kann dieses in einer ersten Anwendungsweise zum Öffnen und Schließen des Maulteiles 56, also zum Erfassen und Klemmen eines Mitralklappensegels 19, wie im nachfolgenden Ablauf dargestellt, benutzt werden. Durch Verschieben des äußeren Rohrschiebers 57 im Trokar 5, z.B. durch Zurückziehen gegenüber dem inneren Rohrschieber I 58, der ortsfest verbleibt, kann das Maulteil 56 des Greifelements 60 geöffnet werden. Das Öffnen des Maulteils 56 geschieht wie folgt: der äußere Rohrschieber 57, der am zylindrischen Rohrelement 25 angedockt ist, zieht die Federarme 50, 50' des Greifelements 60, die über einen Bügel 28 mit dem Rohrelement 25 verbunden sind, weiter in die Öffnung 62 des inneren Rohrschiebers I 58 ein. Beim weiteren Einziehen der Federarme 50, 50' in die Öffnung 62 des inneren Rohrschiebers I 58, werden diese im Bereich des freien Endes 61 weiter zusammengedrückt und der Druck auf die Schenkelfeder 38 wird erhöht, wodurch sich das Maulteil 56 des Greifelementes 60 öffnet, siehe Fig. 2b. Das Prinzip ähnelt einer Wippe bzw. einem zweiseitigen Hebel, der mittig gelagert ist und rechts und links des Drehpunktes zwei, in etwa gleich lange Hebearme aufweist. Wird ein Hebelarm an einem Ende mit einer Gewichtskraft versehen oder wirkt eine Kraft darauf ein, so senkt sich der belastete Arm und der entgegengesetzte bzw. der andere unbelastete Hebelarm bewegt sich in die entgegengesetzte Richtung.

Dieses Prinzip lässt sich auf das Greifelement 60 mit seinen Federarmen 50, 50' übertragen. Jeder Federarm 50, 50' entspricht einem zweiseitigen Hebelarm, daher braucht bei der Beurteilung der Funktionsweise stellvertretend nur ein Federarm 50 betrachtet werden. Ein solcher Federarm 50 weist zwei freie Enden auf. Ein Ende 61 befindet sich an dem geöffneten Greifelement 60 und das andere Ende am Maulteil 56. Ein Federarm 50 ist annähernd mittig an einer Schenkelfeder 38 befestigt, so dass rechts und links der Befestigung annähernd ein gleich langer Hebelarm ausgebildet ist. Wird nun durch den inneren Rohrschieber I 58 eine Kraft auf ein freies Ende 61 eines Federarms 50 (Hebelarm) ausgeübt, so dreht sich der Federarm 50 (Hebelarm) einerseits um den Befestigungspunkt (Drehpunkt) an der Schenkelfeder 38, wodurch sich die beiden freien Enden 61 der Federarme 50, 50' aufeinander zubewegen und sich somit annähern und andererseits wird die Schenkelfeder 38 etwas zusammengedrückt. Das andere Ende des Federarms 50, 50', an welchem das Maulteil 56 angeordnet ist, bewegt sich entgegengesetzt, d.h. voneinander weg und das Maulteil 56 öffnet sich.

Das Maulteil 56 des Greifelements 60 kann aber auch über eine zweite Anwendungsweise der beiden Rohrschieber 57, 58 geöffnet und geschlossen werden, um ein Mitralklappensegel 19 zu erfassen und zu klemmen. Von den beiden, im Vorhof 3 befindlichen Rohrschiebern 57, 58, wird jetzt der äußere Rohrschieber 57 ortsfest gehalten und somit auch das daran angedockte zylindrische Rohrelement 25. Durch Verschieben des inneren Rohrschiebers I 58 in Längsrichtung 53 und längs der Längsachse 33 des Befestigungsmittels 18, kann sich der im inneren des äußeren Rohrschieber 57 und im inneren des zylindrischen Rohrelements 25 befindliche innere Rohrschieber I 58 über die, in der Öffnung 62 befindlichen Federarme 50, 50', weiter erstrecken. Durch das Vorschieben des inneren Rohrschiebers I 58 gegenüber dem äußeren Rohrschieber 57, der mit dem zylindrischen Rohrelement 25 ortsfest verbleibt, kann das Maulteil 56 des Greifelements 60 geöffnet werden. Das Öffnen des Maulteils 56 erfolgt dadurch, dass sich der innere Rohrschieber I 58 über die Federarme 50, 50' des Greifelements 60, die über einen Bügel 28 mit dem Rohrelement 25 verbunden sind, schiebt. Die Federarme 50, 50' können nicht ausweichen, weil diese über die Schenkelfeder 38 mit dem Bügel 28 und dieser wiederum mit dem Rohrelement 25 verbunden sind. Beim weiteren Vorschieben des inneren Rohrschiebers I 58 über die Federarme 50, 50' gleiten diese weiter in die Öffnung 62 des inneren Rohrschiebers I 58. Dabei werden die beiden parallel beabstandeten Federarme 50, 50' weiter zusammengedrückt, wodurch sich das Maulteil 56 des Greifelements 60 öffnet, siehe Fig. 2b.

Das Mitralklappen-Implantat 11 kann mit einem Befestigungsmittel 18 ausgestattet sein. Das Befestigungsmittel 18 soll ein Mitralklappensegel 19, vgl. Fig. 1, erfassen können, wobei die Mitralklappe 6 weiterhin beweglich bleiben muss, aber im Bewegungsspielraum richtungsbegrenzt. Unter Richtungsbegrenzung ist das zurückschlagen des Mitralklappensegels 19 in den Vorhof 3 zu verstehen.

Das Herzklappen-Implantat 11 umfasst zum Beispiel drei Elemente. Ein erstes Element ist das Verankerungselement 13, das als Wendelschraube 14 ausgebildet ist und eine Befestigung des Herzklappen-Implantats 11 im Herzmuskelgewebe 15, siehe Fig. 1, vornimmt. Das zweite Element ist ein Verbindungselement 20, bestehend aus einem künstlichen Faden 21 oder Draht, der die Verbindung zwischen dem Verankerungselement 13 und dem Befestigungsmittel 18 mit Unterstützung eines Klemmmittels 74, herstellt. Diese beiden Elemente wurden bereits in der Fig. 1 aufgezeigt. Ein weiteres Element bildet das Befestigungsmittel 18, welches hier in der Fig. 2a und in der Fig. 2b näher dargestellt wird.

Das Befestigungsmittel 18 selbst kann wiederum mit drei Elementen gebildet sein, zum Beispiel einem Rohrelement 25, einem Verbindungselement 27 und einem Greifelement 60. Das Rohrelement 25 ist vorzugsweise als zylindrische Hülse 25' ausgebildet, wobei die Hülse 25' im Querschnitt geometrische Formen, wie Vierkantrohr usw., aufweisen kann und somit nicht an die Kreisform gebunden ist. Am Außendurchmesser 26 der Hülse 25' sind zwei, sich gegenüberliegende Öffnungen 31, 31' in der Hülsenwand 32 angeordnet. Die Öffnungen 31, 31' befinden sich auf einer Querachse 35, die senkrecht zur Längsachse 33 der Hülse 25' steht, wobei die Öffnungen 31, 31' in Längsrichtung 53 der Hülse 25' betrachtet, ungefähr mittig angeordnet sind. An den Öffnungen 31, 31' und der Außenseite der Hülse 25' ist ein Verbindungselement 27 angeordnet. Das Verbindungselement 27 ist aus einem drahtartigen, annähernd u-förmigen Bügel 28 gebildet, der mit der zylindrischen Hülse 25' in gelenkartiger Verbindung 29 steht. Hierzu weist das freie Ende 30 des u-förmigen Bügels 28 zwei Zapfen (nicht dargestellt) auf, die mindestens um 90 Grad nach innen gebogen sind und wobei jeweils ein Zapfen in eine Öffnung 31, 31' in der Hülsenwand 32 drehbeweglich eingreift. Die Zapfen können auch bis zu 180 Grad nach innen gebogen sein, so dass ein Zapfen parallel zum Längsschenkel 34 verläuft, um somit eine Art Öse zur Aufnahme der Hülsenwand 32 zu bilden. Dadurch einsteht eine nicht lösbare, aber gelenkartige Verbindung 29 zwischen der Hülse 25' und dem Verbindungselement 27. Die Längsschenkel 34 des Bügels 28 weisen eine Länge auf, die wesentlich länger ist als die halbe Hülsenlänge, uzw. in etwa das dreifache der halben Hülsenlänge. Die Länge des Bügels 28 erlaubt dem Bügel 28 daher eine 360 Grad Drehung um die Hülse 25' vollziehen zu können, weil der Querschenkel 36 des Bügels 28, der die beiden Längsschenkel 34, 34' miteinander verbindet, relativ weit von der Hülse 25' entfernt ist. Der Querschenkel 36 bildet zwischen den Längsschenkeln 34, 34' eine durchgehende Verbindungsstange 37, welche Träger eines Greifelementes 60 ist. Das Greifelement 60 kann mit einer Schenkelfeder 38 gebildet sein, die wiederum Träger zweier Federarme 50, 50' ist.

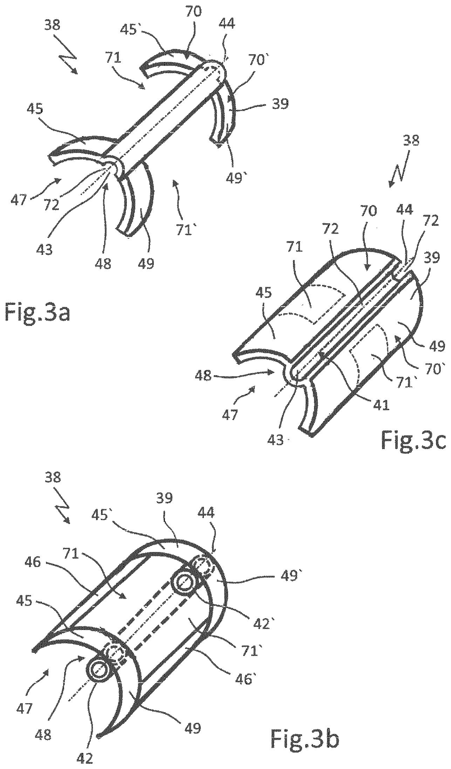

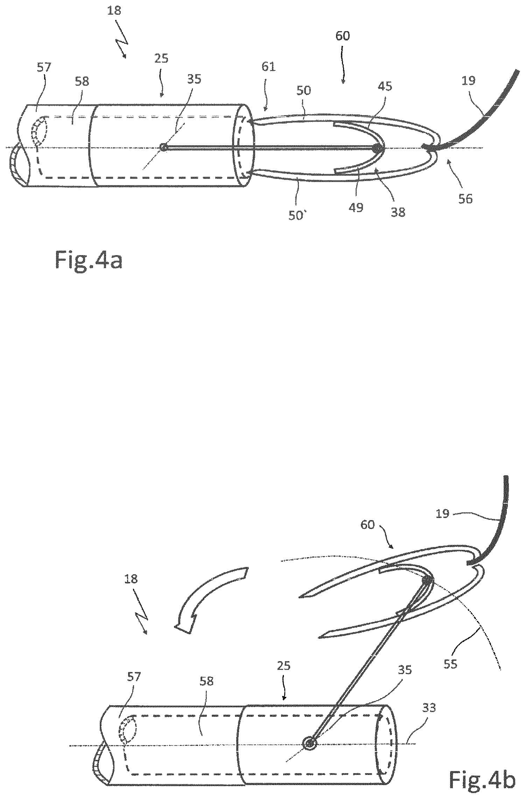

Die in etwa u-förmig ausgebildete Schenkelfeder 38 ist aus einer Blattfeder 39 gebildet, welche im Zentrum des Schenkelbereiches 40 bzw. annähernd im Scheitel 48 der Schenkelfeder 38 ein korrespondierendes Verbindungselement 41, zur Aufnahme einer Verbindungsstange 37 des Bügels 28, aufweist. In den Fig. 3a, 3b und 3c sind verschiedene Ausführungsformen einer Schenkelfeder 38 aufgezeigt. Das Verbindungselement 41 kann aus zwei beabstandeten Ringösen 42, 42' (s. Fig. 3b) bestehen, die fester Bestandteil der Schenkelfeder 38 sind. Die Ringösen 42, 42' befinden sich seitlich am Rand der Schenkelfeder 38 und erstrecken sich nach innen hin, siehe hierzu Fig. 3b. Alternativ kann das Verbindungselement 41 auch aus einem zylindrischen Kanal 43 bzw. einer Längsnut 43 (s. Fig. 3a) bestehen. Der Kanal 43 bzw. die Längsnut 43 verlaufen von der einen Seite bis zur anderen Seite der Schenkelfeder 38 bzw. quer zu den sich erstreckenden Blattfederschenkeln 45, 49, siehe hierzu die Ausführungen in der Fig. 3a. Die Achse 44 der Ringösen 42, 42' der Schenkelfeder 38, gemäß der Ausführungsform der Fig. 3b, oder die Achse 44 des zylindrischen Kanals 43 bzw. der Längsnut 43 der Schenkelfeder 38, gemäß der Ausführungsform der Fig. 3a, verläuft parallel beabstandet zur Querachse 35 der Öffnungen 31, 31' in der Hülse 25' und ebenfalls senkrecht zur Längsachse 33 der Hülse 25'. D.h., die beiden Querachsen 35 und 44 sind parallel beabstandet, wobei die Querachse 44 auf einer Kreisbahn 55 (s. Fig. 4b) um die Querachse 35 schwenken kann. Die Verbindung zwischen der Verbindungsstange 37 des Bügels 28 und dem korrespondierenden Verbindungselement 41 der Schenkelfeder 38 ist derart gestaltet, dass sich die Schenkelfeder 38 um die Verbindungsstange 37 drehbeweglich schwenken lässt. Die u-förmig ausgebildete Schenkelfeder 38 weist mit ihren geöffneten Blattfederschenkeln 45, 49, vom Scheitel 48 ausgehend, in Richtung der zylindrischen Hülse 25' und der Längsschenkel 34, 34' des Bügels 28. Die Blattfederschenkel 45, 45' sind somit annähernd parallel zur Längsachse 33 der Hülse 25' und schwenkbar am Bügel 28 angelenkt und weisen eine Öffnung 47 auf. D.h., die am Bügel 28 angeordnete Schenkelfeder 38 vollzieht mit dem Bügel 28 einerseits eine Drehung um die Querachse 35, welche durch die Hülse 25' führt und andererseits eine Drehung um die Querachse 44, welche durch die Schenkelfeder 38 führt. Die Drehung der Schenkelfeder 38 um die beiden Querachsen 35, 44 kann gleichzeitig erfolgen, ähnlich dem Prinzip einer Gondel beim Riesenrad.

Die Schenkelfeder 38 ist wiederum Träger zweier Federarme 50, 50'. Die zwei Federarme 50, 50' sind mit der Schenkelfeder 38 fest verbunden. Die Verbindung kann durch technisch bekannte Verfahren, wie Lasern, Schweißen, Nieten, Schrauben usw., hergestellt sein. Ein Federarm 50 liegt dabei auf einem Blattfederschenkel 45 und der andere Federarm 50' auf einem gegenüberliegenden Blattfederschenkel 49 auf, sofern die Schenkelfeder 38 nur aus zwei Blattfederschenkeln 45, 49 gebildet ist. Es besteht aber auch die Möglichkeit, wie in der Fig. 3a und Fig. 3b aufgezeigt, dass die Schenkelfeder 38 aus vier Blattfederschenkeln 45, 45', 49, 49' gebildet ist. Das bedeutet, dass der eine Federarm 50 auf den zwei Blattfederschenkeln 45, 45' und der andere Federarm 50' auf den zwei, den Blattfederschenkeln 45, 45' gegenüberliegenden Blattfederschenkeln 49, 49', aufliegt und dort befestigt ist. Die beiden Federarme 50, 50' sind derart an den Blattfederschenkeln 45, 49 bzw. 45, 45' und 49, 49' der Schenkelfeder 38 befestigt, dass diese bei geschlossenem Maulteil 56 auf der Befestigungsseite 63 und mit ihrer gegenüberliegenden Öffnung am freien Ende 61 der Greifmitteiseite 67, annähernd parallel beabstandet sind und sich parallel zur Längsachse 33 der Hülse 25' und somit zur Längsachse 33 des Befestigungsmittels 18 erstrecken. Die beiden zueinander parallel beabstandeten Federarme 50, 50' weisen eine in etwa ovale bzw. konvexe Form, ähnlich einer Sammellinse, auf. D.h., dass die beiden Federarme 50, 50' jeweils nach außen gewölbt sind.

Die Fig. 2c zeigt in schematischer Darstellung das Maulteil 56 eines Greifelementes 60 des Befestigungsmittels 18. Das Maulteil 56 wird aus den Federarmen 50, 50', die Bestandteil der Greifelemente 60 sind, gebildet. Das Maulteil 56 ist an der Befestigungsseite 63 des Greifelementes 60 und auf der abgewandten Seite der geöffneten Schenkelfeder 38 sowie auf der Längsachse 33 des Rohrelements 25 liegend, angeordnet, wobei das Maulteil 56 über mindestens einen, im Maulteil 56 angeordneten Abstandshalter 88 verfügt, der einen vorbestimmten Spalt 89 zwischen den Greifbacken 86, 86' des Maulteils 56 erzeugt, wodurch die Verzahnung 87 der Greifbacken 86, 86' nicht unmittelbar aufeinander zu liegen kommt. Ein Abstandshalter 88 kann z.B. durch die Prägung einer Sicke 90, 90', vorzugsweise von zwei Sicken je Federarm 50, 50', hergestellt werden. Die Einprägung einer Sicke 90, 90' erfolgt auf der Außenseite 91, 91' der Federarme 50, 50' wodurch auf der Innenseite 92, 92' der Federarme 50, 50' eine Erhöhung 93, 93' entsteht. Die Sicken 90, 90' werden in den Federarmen 50, 50' einander gegenüber liegend eingeprägt, so dass die auf der Innenseite 92, 92' erzeugten Erhöhungen 93, 93' beim Schließen des Maulteils 56 aufeinander zu liegen kommen und miteinander korrespondieren und somit einen Abstandshalter 88 bilden. Eine Erhöhung 93, 93' auf der Innenseite 92, 92' zwischen den Federarmen 50, 50' zur Bildung eines Abstandshalters 88, kann auch durch den Einsatz anderer Mittel erfolgen.

Die Fig. 3a, 3b und 3c zeigen in perspektivischer Ansicht jeweils eine Ausführungsform einer Schenkelfeder 38, die Bestandteil des Greifelements 60 ist. Gemäß der Fig. 2a und 2b besteht die Ausführungsform des Greifelements 60 aus zwei gleichen, länglichen Federarmen 50, 50' aus nicht rostendem, legiertem Metall das bestimmte elastische Eigenschaften aufweist und vorzugsweise aus Nitinol bestehen kann. Die Federarme 50, 50' sind in der Mitte an einer Schenkelfeder 38 aus nicht rostendem Metall befestigt und werden somit zusammengehalten. Die Schenkelfeder 38 dient gleichzeitig als Gelenk. Durch Zusammendrücken der beiden Federarme 50, 50' und somit der beiden Blattfederschenkel 45, 49 der Schenkelfeder 38 an einem Ende 61 der Federarme 50, 50' mit Hilfe eines inneren Rohrschiebers I 58, öffnet sich das Maulteil 56 am anderen Ende der Befestigungsseite 63, s. Fig. 2b. Werden die Federarme 50, 50' wieder vom Rohrschieber I 58 freigegeben, drückt die, der Schenkelfeder 38 innewohnende Kraft, die Federarme 50, 50' auf der einen Seite des freien Endes 61 auseinander und auf der anderen Seite des Maulteils 56 wieder zusammen, s. Fig. 2a u. Fig. 4a. Das Greifelement 60 arbeitet somit nach dem Wäscheklammerprinzip.

Das einstückige Greifelement 60 besteht aus einer Schenkelfeder 38 mit fest daran angeordneten Federarmen 50, 50', wie aus den Fig. 2a und 2b ersichtlich. Zur besseren Darstellung und Übersichtlichkeit einer einstückigen Schenkelfeder 38 und deren unterschiedlichen Ausführungsformen, wurde auf die Darstellung der Federarme 50, 50' in den Fig. 3a, 3b, 3c, verzichtet, obwohl diese natürlich Gegenstand an einer Schenkelfeder 38 und somit des Greifelements 60 sind. Die in den Fig. 2a und 2b in Bezug auf das Greifelement 60 und die Schenkelfeder 38 identisch aufgezeigten Bezugszeichen werden hier analog übernommen.

Alle Ausführungsformen von Schenkelfedern 38 ist gemeinsam, dass die Basis aus einer Blattfeder 39 besteht. Diese Blattfedern 39 sind aus elastischem Federstahl hergestellt und u-förmig ausgebildet und weisen daher zwischen ihren gebogenen Schenkeln eine Öffnung 47 auf. Die u-förmig ausgebildeten Blattfedern 39 bilden daher jeweils zwei Blattfederschenkel 45, 49. Die Flächen der Blattfederschenkel 45, 49 können in ihrer Fläche 70, 70' Ausnehmungen 71, 71' aufweisen, wobei die Ausnehmungen 71, 71' in der Fläche 70, 70' offen oder geschlossen sein können. Aufgrund der Ausnehmungen 71, 71' können mehrere Blattfederschenkel 45, 45', 49, 49' entstehen. Eine weitere Gemeinsamkeit der Schenkelfedern 38 besteht darin, dass eine Schenkelfeder 38 in ihrem Scheitel 48 ein korrespondierendes Verbindungselement 41, zur Aufnahme einer Verbindungsstange 37 eines Bügels 28 (s. Fig. 2a und 2b), aufweist. Beispielsweise kann das Verbindungselement 41 aus zwei beabstandeten Ringösen 42, 42' (s. Fig. 3b) bestehen, die fester Bestandteil der Schenkelfeder 38 sind. Die Ringösen 42, 42' befinden sich seitlich am Rand der Schenkelfeder 38 und erstrecken sich in Querrichtung der Schenkelfeder 38 nach innen und weisen eine Querachse 44 auf. Die Ringösen 42, 42' der u-förmigen Schenkelfeder 38 befinden sich auf der Innenseite der Öffnung 47 der, der Blattfederschenkel 45, 49 zugewandten Seite. Alternativ können die Ringösen 42, 42' im Scheitelbereich 48 der Schenkelfeder 38 aber auf der Außenseite der Blattfederschenkel 45, 49, nicht dargestellt, also der, der Öffnung 47 abgewandten Seite, angeordnet sein. Die Ringösen 42, 42' der Schenkelfeder 38 sind derart ausgebildet, das sie mit der Verbindungsstange 37 eines Bügels 28 eine gelenkartige Verbindung 29' ergeben.

Alternativ kann das Verbindungselement 41 einer Schenkelfeder 38 auch aus einem zylindrischen Kanal 43 bzw. einer Längsnut 43 (s. Fig. 3a) bestehen. Der Kanal 43 bzw. die Längsnut 43 verlaufen von der einen Querseite bis zur anderen Querseite der Schenkelfeder 38 bzw. quer zu den sich erstreckenden Blattfederschenkeln 45, 49. Der zylindrische Kanal bzw. die Längsnut 43 ist derart gestaltet, das der zylindrische Kanal bzw. die Längsnut 43 einen Schlitz 72 aufweist, der in Richtung der aufeinander zuweisenden Blattfederschenkel 45, 49 geöffnet ist. Der Schlitz 72 ist durchgehend geöffnet, um die Verbindungsstange 37 eines Bügels 28 aufnehmen zu können. Der zylindrische Kanal bzw. die Längsnut 43 der Schenkelfeder 38 ist derart ausgebildet, dass diese mit der Verbindungsstange 37 eines Bügels 28 eine feste, aber gelenkige Verbindung 29' ergeben. Die Längsnut 43 weist eine Querachse 44 auf, die identisch mit der Querachse 44 der Ringösen 42, 42' verläuft. Auch dieses Verbindungselement 41 befindet sich mit seinem zylindrischen Kanal oder der Längsnut 43 auf der Innenseite der Öffnung 47 der, den Blattfederschenkeln 45, 49 zugewandten Seite. Die Blattfederschenkel 45, 49 bilden mit dem zylindrischen Kanal bzw. der Längsnut 43 eine einstückige Schenkelfeder 38.

In einer weiteren Ausführungsform einer Schenkelfeder 38, s. Fig. 3c, befindet sich das Verbindungselement 41 eines zylindrischen Kanals bzw. einer Längsnut 43 nicht im Öffnungsbereich 47 der Blattfederschenkel 45, 49 bzw. nicht auf der Innenseite zwischen den Blattfederschenkeln 45, 49, sondern auf der Außenseite der Blattfederschenkel 45, 49, aber trotzdem im Bereich des Scheitels 48. Natürlich ist die Querachse 44 dieser Ausführungsform von Schenkelfeder 38 identisch mit den Querachsen der Schenkelfedern 38 aus den Fig. 3a und 3b. Auch diese Schenkelfeder 38 ist einstückig ausgebildet und weist einen Schlitz 72 zur Aufnahme der Verbindungsstange 37 eines Bügels 28 auf. Der zylindrische Kanal bzw. die Längsnut 43 der Schenkelfeder 38 ist derart ausgebildet, das diese mit der Verbindungsstange 37 eines Bügels 28 eine feste, aber gelenkige Verbindung 29' ergeben.

Die Fig. 4a und 4b zeigen in schematischer Darstellung ein Befestigungsmittel 18 mit, im Greifelement 60 eingeklemmtem Mitralklappensegel 19. Gemäß der Fig. 4a, erfolgt der Vorgang des Erfassens und Klemmens eines Mitralklappensegels 19 im linken Vorhof 3 des Herzens 1, wobei der Vorgang des Öffnens und Schließens des Maulteils 56 am Greifelement 60 bereits in der Fig. 2a und 2b beschrieben wurde. Die Funktionsweise des Greifelements 60 geht aus den Fig. 3a-3c hervor. Grundsätzlich wird vor dem Ergreifen eines Mitralklappensegels 19 das Greifelement 60 geöffnet und mit geöffnetem Maulteil 56 ein Mitralklappensegel 19 erfasst. Zur Erfassung eines Mitralklappensegels 19 werden beide Rohrschieber 57, 58, die das Befestigungsmittel 18 transportieren und bedienen, in Richtung Mitralklappe 6 vorgeschoben. Das Verschieben des Befestigungsmittels 18 wird mit Hilfe von bekannten Geräte basierenden bildgebenden Verfahren beobachtet. Befindet sich das geöffnete Maulteil 56 des Greifelements 60 in der richtigen Position zum Mitralklappensegel 19, wird das Maulteil 56 geschlossen. Das Schließen des Maulteils 56 erfolgt durch das Zurückziehen des inneren Rohrschiebers I 58 bei gleichzeitigem Festhalten des äußeren Rohrschiebers 57, wodurch das zylindrische Rohrelement 25 ortsfest verbleibt. Der innere Rohrschieber I 58 kann jetzt entfernt werden. Beim Entfernen des Rohrschiebers I 58 entspannt sich die Schenkelfeder 38 und die beiden Blattfederschenkel 45, 49 drücken die beiden Federarme 50, 50' an den freien Enden 61 eine bestimmte Weglänge auseinander. An dem gegenüberliegenden Ende der Federarme 50, 50', an dem das Maulteil 56 angeordnet ist, wirkt jetzt die maximale Kraft der Schenkelfeder 38 auf das Maulteil 56 ein, dadurch wird das, im Maulteil 56 befindliche Mitralklappensegel 19, geklemmt. Die Klemmung erfolgt, wie in der Fig. 2c beschrieben.

Im nächsten Schritt erfolgt das Schwenken des Greifelements 60 um die Querachse 35 des zylindrischen Rohrelements 25 und das Einführen des Befestigungsmittels 18 in den linken Ventrikel 7, um dieses einerseits dort zu positionieren und andererseits im Herzmuskelgewebe 15 zu befestigen. Dieser Vorgang wird in der Fig. 4b und 4c aufgezeigt.

Aus der Fig. 4b ist, in schematischer Darstellung, ein Befestigungsmittel 18 mit eingeklemmtem Mitralklappensegel 19 beim Schwenkvorgang des Greifelementes 60 ersichtlich. Um das Befestigungsmittel 18, welches bereits ein Mitralklappensegel 19 erfasst und geklemmt hat, vom linken Vorhof 3, (d. h., das Befestigungsmittel 18 befindet sich noch oberhalb der Mitralklappe 6) in den linken Ventrikel 7 unterhalb der Mitralklappe 6 zu befördern, ist es notwendig, mit dem äußeren Rohrschieber 57 ein weiteres Handling bzw. Vorschieben des äußeren Rohrschiebers 57 durchzuführen (s. u.a. in der Fig. 1). Hierzu führt der äußere Rohrschieber 57 zuerst das lösbar an ihm befestigte zylindrische Rohrelement 25 und danach das am Rohrelement 25 angeordnete Greifelement 60 durch die Ventilöffnung 94 in der Mitralklappe 6 in den linken Ventrikel 7 ein. Der Schwenkvorgang des Greifelements 60 um das Rohrelement 25 ist beendet, wenn das Greifelement 60 außen am äußeren Rohrschieber 57 zum Anliegen kommt. In dieser Position befindet sich das, am Befestigungsmittel 18 schwenkbar angeordnete Greifmittel 60, mit geklemmtem Mitralklappensegel 19 unterhalb der Mitralklappe 6 im linken Ventrikel 7, wie sich aus der Fig. 1 und 5 ergibt. Auch dieses Handling, das Verschieben des Befestigungsmittels 18 mit Hilfe des äußeren Rohrschiebers 57 in den linken Ventrikel 7, wird mit Hilfe von bekannten bildgebenden Verfahren kontrolliert. Die Kontrolle ist maßgebend bei der Führung des äußeren Rohrschiebers 57.

Das Befestigen des Befestigungsmittels 18 im Herzmuskelgewebe 15 des linken Ventrikels 7 wird in schematischer Darstellung in der Fig. 4c und das Positionieren des Herzklappen-Implantats 11 in der Fig. 5 aufgezeigt.

Aus der Fig. 4c ist in schematischer Darstellung ein chirurgisches Instrument zur Führung und Befestigung des, am Verbindungselement 20 angeordneten Verankerungselementes 13 im Herzmuskelgewebe 15, ersichtlich. Das chirurgische Instrument wird hier als innerer Rohrschieber II 59 bezeichnet. Der innere Rohrschieber II 59 ist im Inneren mit einem Verbindungselement 20 und einem Verankerungselement 13 bestückt, wobei das Verbindungselement 20 mit dem Verankerungselement 13 fest verbunden ist. Das Verbindungselement 20 besteht aus einem Faden 21, der aus Polytetrafluorethylen (PTFE) hergestellt ist. Das eine (zweite) Ende des Fadens 21 des Verbindungselementes 20 befindet sich außerhalb vom Thorax des Patienten, während das andere (erste) Ende 24 des Fadens 21 mit dem proximalen Ende 51 des Verankerungselementes 13 fest verbunden ist. Das Verankerungselement 13 besteht aus einer Wendelschraube 14, die aus einer Nickel-Titan-Legierung, vorzugsweise aus Nitinol hergestellt sein kann. Zur Führung und Befestigung des wendelförmigen Verankerungselements 13 weist der innere Rohrschieber II 59 am Einführende 73 ein Klemmmittel 74 auf. Das Klemmmittel 74 kann als Wendelnute 75 in der Rohrwand 76 ausgebildet sein, wobei die Wendelnute 75 eine Kurve bildet, die mit konstanter Steigung in der Wandung des inneren Rohrschiebers II 59 angeordnet ist. Die Steigung und Gängigkeit der Wendelnute 75 korrespondiert mit der Steigung der rechtsgängigen Wendelschraube 14. Die Wendelnute 75 ist relativ kurz am Einführende 73 des inneren Rohrschiebers II 59 ausgebildet und erfasst beim Drehen des inneren Rohrschiebers II 59 im Uhrzeigersinn die Wendelschraube 14 am proximalen Ende 51.

Bereits vor dem Einschieben des inneren Rohrschiebers II 59 durch einen Trokar 5 und durch die Bohrung 66 (Innendurchmesser) des Befestigungsmittels 18 in den linken Ventrikel 7, wurde das Verbindungselement 20 und das Verankerungselement 13 in das Innere des Rohrschiebers II 59 eingebracht. Das Verankerungselement 13 befindet sich am Einführende 73 des Rohrschiebers II 59, wobei sich das proximale Ende 51 der Wendelschraube 14 in der Wendelnute 75 befindet. Die Auswahl der Größe der zu verwendenden Wendelschraube 14 wurde vor der Operation ermittelt, um auf die unterschiedliche Dicke des Herzmuskelgewebes 15 im Bereich des Apex 16 zu reagieren. Es stehen also unterschiedliche Längen von Wendelschrauben 14 zur Verfügung. Das distale Ende 52 der Wendelschraube 14 wird jetzt durch Rechtsdrehung des Rohrschiebers II 59 in das Herzmuskelgewebe 15 eingebracht. Ist die Endposition der Wendelschraube 14 im Herzmuskelgewebe 15 erreicht, wird durch einfache Linksdrehung des Rohrschiebers II 59 die Verbindung zur Wendelschraube 14 gelöst. Das proximale Ende 51 der Wendelschraube 14 gleitet bei der Linksdrehung des Rohrschiebers II 59 aus der Wendelnute 75 heraus. Der innere Rohrschieber II 59 wird jetzt durch Rückwärtsziehen aus dem Befestigungsmittel 18 und dem Trokar 5 entfernt.

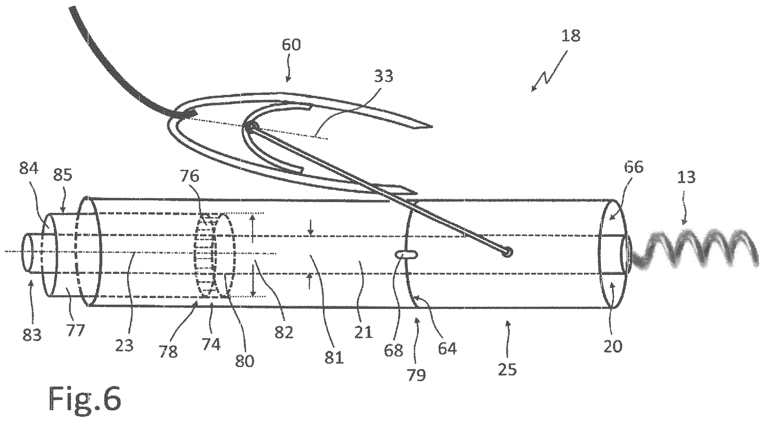

Wie aus der Fig. 5, in schematischer Darstellung ersichtlich, befindet sich im linken Ventrikel 7 des Herzens 1 jetzt ein Herzklappen-Implantat 11, wobei das Befestigungsmittel 18 ein Mitralklappensegel 19 erfasst hat und das spiralförmige Verankerungselement 13 im Herzmuskelgewebe 15 verankert ist. Der Faden 21 des Verbindungselementes 20 verläuft derzeit noch vom Verankerungselement 13 durch das zylindrische Rohrelement 25 eines Befestigungsmittels 18, durch einen am Befestigungsmittel 18 angedockten äußeren Rohrschieber 57 und von dort durch den, im linken Vorhof 3 eingesetzten Trokar 5 bis außerhalb des Thorax. Die beiden inneren Rohrschieber I, II 58, 59 wurden bereits vorher entfernt. Z. Z. befindet sich nur noch der äußere Rohrschieber 57, wie aus der Fig. 6 ersichtlich, am Befestigungsmittel 18 im Einsatz. Zur Führung des äußeren Rohrschiebers 57 wird weiterhin der eingesetzte Trokar 5 benutzt. Zur Konfektionierung des Herzklappen-Implantats 11 ist es notwendig, noch eine Verbindung zwischen dem Verbindungselement 20 und dem Befestigungsmittel 18 herzustellen. Die Verbindung wird in der Fig. 6 aufgezeigt, wobei Bezugszeichen aus den vorgenannten Figuren darin aufgeführt sein können.

Die Fig. 6 zeigt in schematischer Darstellung die Herstellung der Verbindung zwischen einem Befestigungsmittel 18 und einem Verankerungselement 13 mit Hilfe des Verbindungselements 20 und einem Klemmmittel 74. Das Verbindungselement 20 besteht aus einem Faden 21, der vom Verankerungselement 13 bis außerhalb des Thorax reicht und durch die Innenöffnung 66 des Befestigungsmittels 18 führt. Auf das außen liegende Ende 83 des Fadens 21 wird ein Klemmmittel 74 aufgeschoben. Zum Schieben des Klemmmittels 74 auf dem Faden 21 wird erneut ein innerer Rohrschieber III 77 benutzt. Der innere Rohrschieber III 77 nimmt in seiner Bohrung 84 einerseits einen Faden 21 und ein Klemmmittel 74 auf. Andererseits passt der Außendurchmesser 85 des inneren Rohrschiebers III 77 in den äußeren Rohrschieber 57. Mit der Stirnseite 78 des Rohrschiebers III 77 wird das Klemmmittel 74 auf dem Faden 21 entlang der Längsachse 23 zur Befestigungsstelle 79 geschoben. Die Befestigungsstelle 79 befindet sich an der Flanschseite 64, am Eingang der Innenöffnung 66 des zylindrischen Rohrelements 25. Das Material des Klemmmittels 74 kann vorzugsweise aus PTFE bestehen, auch andere Materialien sind denkbar. Die Bohrung 80 im Klemmmittel 74 ist dem Durchmesser 81 des Fadens 21 in etwa angepasst, mit der Maßgabe, dass der Faden 21 im Klemmmittel 74 schwergängig bzw. das Klemmmittel 74 nur mit einer bestimmten Kraft auf dem Faden 21 verschoben werden kann. Die Bohrung 80 und der Durchmesser 81 bilden eine Presspassung. Gleiches trifft auf den Außendurchmesser 82 des Klemmmittels 74 und den Innendurchmesser 66 des zylindrischen Rohrelements 25 zu. Der Außendurchmesser 82 ist geringfügig größer als der Innendurchmesser 66, wodurch sich wiederum eine Presspassung ergibt. Zum Einführen des Klemmmittels 74 in das Befestigungsmittel 18 sind der Faden 21 des Verbindungselements 20 und die Bohrung 84 des inneren Rohrschiebers III 77 aufgrund der Presspassungen mit einem Gleitmittel versehen. Das Klemmmittel 74 kann unterschiedliche Ausführungsformen umfassen. Bei einer Ausführungsform des Klemmmittels 74 ist dieses zylindrisch als Gleitring 76 ausgebildet. In einer anderen Ausführungsform ist das Klemmmittel 74 kegelstumpfartig, ähnlich einem Flaschenkorken (nicht dargestellt), ausgebildet. Der kleinere Durchmesser des Kegelstumpfs, der geringfügig kleiner ist als die Innenöffnung 66 des zylindrischen Rohrelements 25, wird mit Hilfe des Rohrschiebers III 77 zuerst eingeschoben. Der Klemmeffekt tritt auf der Mantelfläche des Kegelstumpfes ein. Die Positionierung des Befestigungsmittels 18 im linken Ventrikel 7 erfolgt, z.B. mit Hilfe des Gleitrings 76. Der Gleitring 76 wird in der optimalen Position des Befestigungsmittels 18 zur Mitralklappe 6 in das zylindrische Rohrelement 25 eingeschoben. Die Ermittlung der optimalen Position des Befestigungsmittels 18 erfolgt wieder mit den bekannten bildgebenden Messverfahren, wobei auch der Blutrückfluss während der Kontraktion des linken Ventrikels in den linken Vorhof 3 ermittelt wird. Die optimale Position ist erreicht, wenn der Rückfluss des Blutes am minimalsten ist. Ist die optimale Position noch nicht erreicht, wird der Gleitring 76 auf dem Faden 21 entlang der Längsachse 23 in Richtung des Verankerungselements 13 mit dem Rohrschieber III 77 weiter verschoben. Während des Verschiebens des Gleitrings 76 auf dem Faden 21 wird einerseits der Faden 21 außerhalb des Thorax fixiert und das Befestigungselement 18 mit dem äußeren Rohrschieber 57 ortsfest gehalten. Nach dem erfolgreichen Platzieren des Gleitrings 76 im Befestigungsmittel 18 ist das Herzklappen-Implantat 11 komplett konfektioniert und der innere Rohrschieber III 77 kann zurückgezogen und entfernt werden. Der Rohrschieber III 77 wird jetzt durch ein bekanntes chirurgisches Instrument (nicht dargestellt) ersetzt. Mit dem chirurg. Instrument kann der Faden 21 am Gleitring 76 verknotet und abgeschnitten werden, wobei auch andere Befestigungsarten denkbar sind. Nach dem Entfernen des restlichen Fadens 21 aus dem Herzen 1 und dem Thorax sowie dem chirurg. Instrument, wird auch der äußere Rohrschieber 57 vom Befestigungsmittel 18 getrennt. Hierzu wird der äußere Rohrschieber 57 aus der Verbindungsstelle 68 gelöst, z.B. durch Drehen, je nachdem wie die Verbindungstelle 68 ausgestaltet ist. Nach dem Entfernen des äußeren Rohrschiebers 57 aus der Ventilöffnung der Mitralklappe 6, schwenkt das Greifelement 60 auf die Längsachse 23 des Herzklappen-Implantats 11. Die Längsachse 33 des Greifelements 60 und des Befestigungsmittels 18 ist jetzt identisch mit der Längsachse 23 des Verbindungselements 20 und dem Verankerungselement 13. Beide Längsachsen 23, 33 bilden jetzt eine gemeinsame Achse. Das Einsetzen eines erfinderischen Herzklappen-Implantats 11 in den linken Ventrikel 7 eines Herzens 1 ist damit beendet.

Das vorangegangene Ausführungsbeispiel, gemäß der Fig. 1 bis 6, zeigt ein Mitralklappen-Implantat, das im linken Ventrikel konfektioniert und, relativ zu einem Mitralklappensegel eingesetzt, eine Regurgitation beseitigt. Die Regurgitation ist ein Vorgang, bei dem der Inhalt aus dem Hohlorgan des Herzens nicht nur den üblicherweise vorgesehenen Weg nimmt, sondern teilweise oder überwiegend zurück in die andere Richtung fließt. Dieser Vorgang ist beim Menschen und bei vielen Tierarten krankhaft. Zur Beseitigung einer Regurgitation sind zusätzliche Ausführungsformen beim Herzklappen-Implantat, insbesondere bei dem Befestigungsmittel des Herzklappen-Implantats, möglich und daher nicht auf das Ausführungsbeispiel begrenzt. Dieses betrifft auch auf die Verbindungstechnik zwischen dem Verbindungselement und dem Befestigungsmittel zu.

**Bezugszeichenliste**

| | | | |
|---|---|---|---|
| 1 | Herz | 30 | Freies Ende (v.28) |
| 2 | Linke Herzkammer | 31,31' | Öffnung |
| 3 | Linker Vorhof | 32 | Hülsenwand |
| 4 | Zugang | 33 | Längsachse (v.25, 25') |
| 5 | Trokar | 34,34' | Längsschenkel (v.28) |
| 6 | Ventil-/Mitralklappe | 35 | Querachse |
| 7 | Linkes Ventrikel | 36 | Querschenkel |
| 8 | Sehnenfäden | 37 | Verbindungsstange |
| 9 | Papillarmuskeln | 38 | Schenkelfeder (v.60) |
| 10 | Ventrikelwand | 39 | Blattfeder |
| 11 | Mitralklappen-Implantat | 40 | Schenkelbereich |
| 12 | Distales Ende (v.11) | 41 | Verbindungselement |
| 13 | Verankerungselement | 42,42' | Ringösen |
| 14 | Wendelschraube | 43 | Kanal/Längsnut |
| 15 | Herzmuskelgewebe | 44 | Querachse |
| 16 | Herzbereich (Apex) | 45,45' | Blattfederschenkel |
| 17 | Proximales Ende (v.11) | 46,46' | Querstrebe |
| 18 | Befestigungsmittel | 47 | Öffnung Blattfederschenkel |
| 19 | Mitralklappensegel | 48 | Scheitel |
| 19.1 | Vorderes Klappensegel | 49,49' | Blattfederschenkel |
| 19.2 | Hinteres Klappensegel | 50,50' | Federarme |
| 20 | Verbindungselement | 51 | Proximales Ende (v.14) |
| 21 | Faden | 52 | Distales Ende (v.14) |
| 22 | Gerissener Sehnenfaden | 53 | Längsrichtung |
| 23 | Längsachse (v.11) | 54 | Ende (v.28) |
| 24,24' | Erstes-, zweites Ende | 55 | Kreisbahn |
| 25,25' | Rohrelement/Hülse | 56 | Maulteil |
| 26 | Außendurchmesser | 57 | Äußerer Rohrschieber |
| 27 | Verbindungselement zw.25,38) | 58 | Innerer Rohrschieber I |
| 28 | Bügel | 59 | Innerer Rohrschieber II |
| 29,29' | Gelenkartige Verbindung | 60 | Greifelement |
| 61 | Freies Ende (v.60) | 90,90' | Sicke (in 50, 50') |
| 62 | Öffnung (in 58) | 91,91' | Außenseite ( v.50, 50') |
| 63 | Befestigungsseite (v.18, 60) | 92,92' | Innenseite ( v.50, 50') |
| 64 | Flanschseite (v.18, 25) | 93,93' | Erhöhung |
| 65 | Andockseite (v.25) | 94 | Ventilöffnung |
| 66 | Innendurchmesser/Öffn. (v.25) | | |
| 67 | Greifarmseite | | |
| 68 | Verbindungsstelle (zw.25,57) | | |
| 69 | Innendurchmesser (v.58) | | |
| 70,70' | Fläche / Außenseite | | |
| 71 | Ausnehmung | | |
| 72 | Schlitz | | |
| 73 | Einführende | | |
| 74 | Klemmmittel | | |
| 75 | Wendelnute | | |
| 76 | Gleitring | | |
| 77 | Rohrschieber | | |
| 78 | Stirnseite | | |
| 79 | Befestigungs-Verbindungsstelle | | |
| 80 | Bohrung (v.76) | | |
| 81 | Durchmesser (v.21) | | |
| 82 | Außendurchmesser (v.76) | | |
| 83 | Fadenende | | |
| 84 | Bohrung (v.77) | | |
| 85 | Außendurchmesser (v.77) | | |
| 86,86' | Greifbacken (v.56) | | |
| 87 | Verzahnung | | |
| 88 | Abstandshalter | | |
| 89 | Spalt (v.56) | | |

## Patentansprüche

1. Herzklappen-Implantat (11) zur minimalinvasiven Reparatur einer Ventilklappe (6) im schlagenden Herzen (1) eines Patienten, mit
- einem Verbindungselement (20), wobei das Verbindungselement (20) mit einem ersten (24) und einem zweiten Ende (24'), im Allgemeinen einander gegenüberliegend, ausgestattet ist,
- einem Verankerungselement (13), das ein proximales (51) und ein distales Ende (52) aufweist, wobei das proximale Ende (51) an dem ersten Ende (24) des Verbindungselementes (20) angeordnet ist und am zweiten Ende (24') des Verbindungselementes (20) ein Befestigungsmittel (18) angeordnet ist, und
- ein Klemmmittel (74), welches das Verbindungselements (20) mit dem Befestigungselement (18) verbindet,
**dadurch gekennzeichnet, dass** das Befestigungsmittel (18) Folgendes aufweist:
- ein Rohrelement (25);
- ein Verbindungselement (27) des Befestigungsmittels (18), wobei das Verbindungselement (27) einen Bügel (28) mit einem freien Ende (30) aufweist, welches schwenkbar in einem Rohrelement (25) des Befestigungsmittels (18) angeordnet ist; und
- ein Greifelement (60), wobei
- das Verbindungselement (27) des Befestigungsmittels (18) ein freies Ende (30) aufweist, welches schwenkbar in dem Rohrelement (25) angeordnet ist, und
- am anderen, in Längsrichtung (53) der Längsachse (33) gegenüberliegenden Ende (54) des schwenkbaren Verbindungselements (27) des Befestigungsmittels (18), ist eine Schenkelfeder (38) schwenkbar angeordnet ist, an der, wiederum fest mit dieser verbunden, zwei durch die Schenkelfeder (38) parallel beabstandete Federarme (50, 50') angeordnet sind.

2. Herzklappen-Implantat (11) nach Anspruch 1, **dadurch gekennzeichnet, dass** das Rohrelement (25) mit dem Verbindungselement (27) des Befestigungsmittels (18) eine gelenkartige Verbindung (29) und das Verbindungselement (27) des Befestigungsmittels (18) mit dem Greifelement (60) eine gelenkartige Verbindung (29') bildet.

3. Herzklappen-Implantat (11) nach Anspruch 1 bis 2, **dadurch gekennzeichnet, dass** das Rohrelement (25) als zylindrische Hülse (25') ausgebildet ist und zwei sich gegenüberliegende Öffnungen (31, 31') in der Hülsenwand (32), auf einer Querachse (35) liegend, aufweist, wobei die Querachse (35) senkrecht zur Längsachse (33) der Hülse (25') steht und die Öffnungen (31, 31'), in Längsrichtung der Hülse (25') betrachtet, mittig in der Hülsenwand (32) angeordnet sind.

4. Herzklappen-Implantat (11) nach Anspruch 1, **dadurch gekennzeichnet, dass** das Verbindungselement (27) des Befestigungsmittels (18) einen Bügel (28) bildet, der zwei Längsschenkel (34, 34') und einen Querschenkel (36) umfasst, wobei an den freien Enden (30) der Längsschenkel (34, 34') ein Zapfen oder eine Öse angeordnet sind und der Querschenkel (36) eine Verbindungsstange (37) mit Querachse (44) bildet, die zur Querachse (35) in der Hülse (25') parallel beabstandet ist und als Verbindungsstange (37) Träger einer Schenkelfeder (38) ist.

5. Herzklappen-Implantat (11) nach Anspruch 1, **dadurch gekennzeichnet, dass** die Schenkelfeder (38) des Greifelements (60) aus einer Blattfeder (39) u-förmig ausgebildet ist und mindestens zwei Blattfederschenkel (45, 45', 49, 49') aufweist, wobei im innenliegenden Scheitel (48) des Schenkelbereichs (40) ein zur Verbindungsstange (37) des Bügels (28) korrespondierendes Verbindungselement (41) mit Querachse (55) angeordnet ist.

6. Herzklappen-Implantat (11) nach Anspruch 5, **dadurch gekennzeichnet, dass** das Verbindungselement (41) aus mindestens einer Ringöse (42, 42'), einem zylindrischen Kanal (43), einer Längsnut (43) oder einem Schlitz (72) gebildet ist.

7. Herzklappen-Implantat (11) nach Anspruch 5 bis 6, **dadurch gekennzeichnet, dass** das Verbindungselement (41) auf der Außenseite (70) der Blattfederschenkel (45, 45', 49, 49') im Schenkelbereich (48) der abgewandten Seite der Öffnung (47) der Blattfederschenkel (45, 45', 49, 49') angeordnet ist.

8. Herzklappen-Implantat (11) nach Anspruch 5, **dadurch gekennzeichnet,dass** die Blattfeder (39) der Schenkelfeder (38) in der Fläche (70) mindestens eine Ausnehmung (71) im Blattfederschenkel (45, 49) aufweist.

9. Herzklappen-Implantat (11) nach Anspruch 1, **dadurch gekennzeichnet, dass** das Greifelement (60) ein, durch die Federarme (50, 50') gebildetes Maulteil (56) aufweist, das an der Befestigungsseite (63) des Greifelementes (60) und auf der abgewandten Seite der geöffneten Schenkelfeder (38) sowie auf der Längsachse (33) des Rohrelements (25) liegend angeordnet ist, wobei das Maulteil (56) über mindestens einen, im Maulteil (56) angeordneten Abstandshalter (88) verfügt, der einen vorbestimmten Spalt (89) zwischen der Verzahnung (87) der Greifbacken (86) im Maulteil (56) erzeugt.

10. Herzklappen-Implantat-System zur minimal-invasiven Reparatur einer Ventilklappe (6) im schlagenden Herzen (1) eines Patienten, mit
- einem äußeren Rohrschieber (57) mit Lumen zum Führen und Halten eines Befestigungsmittels (18);
- einem ersten inneren Rohrschieber I (58) mit Lumen zum Öffnen und Schließen eines Greifelementes (60);
- einem zweiten inneren Rohrschieber II (59) mit Lumen zum Führen und Eindrehen eines Verankerungselements (13);
- einem dritten inneren Rohrschieber III (77) zum Einführen und Positionieren eines Gleitrings (76); und
- ein Herzklappen-Implantat (11), aufweisend:
- ein Verbindungselement (20), wobei das Verbindungselement (20) mit einem ersten (24) und einem zweiten Ende (24'), im Allgemeinen einander gegenüberliegend, ausgestattet ist;
- ein Verankerungselement (13), das ein proximales (51) und ein distales Ende (52) aufweist, wobei das proximale Ende (51) am ersten Ende (24) des Verbindungselementes (20) angeordnet ist und am zweiten Ende (24') des Verbindungselementes (20) ein Befestigungsmittel (18) angeordnet ist;
- ein Befestigungsmittel (18), welches ein Rohrelement (25) aufweist;
- ein Klemmmittel (74), welches das Verbindungselements (20) mit dem Befestigungselement (18) verbindet; und
- ein Verbindungselement (27) des Befestigungsmittels (18);
wobei
- das Verbindungselement (27) des Befestigungsmittels (18) einen Bügel (28) aufweist, der ein freies Ende (30) aufweist, welches schwenkbar in dem Rohrelement (25) angeordnet ist,
- am anderen, in Längsrichtung (53) der Längsachse (33) gegenüberliegenden Ende (54) des schwenkbaren Verbindungselements (27) des Befestigungsmittels (18) ein Greifelement (60) schwenkbar angeordnet ist, und
- das Greifelement (60) eine Schenkelfeder (38) aufweist, an der, fest mit dieser verbunden, zwei durch die Schenkelfeder (38) parallel beabstandete Federarme (50, 50') angeordnet sind.

11. Herzklappen-Implantat-System nach Anspruch 10, **dadurch gekennzeichnet, dass** das Greifelement (60) ein durch die Federarme (50, 50') gebildetes Maulteil (56) aufweist, das an der Befestigungsseite (63) des Greifelementes (60) und auf der abgewandten Seite der geöffneten Schenkelfeder (38) sowie auf der Längsachse (33) des Rohrelements (25) liegend angeordnet ist, wobei das Maulteil (56) über mindestens einen, im Maulteil (56) angeordneten Abstandshalter (88) verfügt, der einen vorbestimmten Spalt (89) zwischen der Verzahnung (87) der Greifbacken (86) im Maulteil (56) erzeugt.

## Claims

1. A heart valve implant (11) for the minimally invasive repair of a valve flap (6) in the beating heart (1) of a patient, with
- a connecting element (20), wherein the connecting element (20) is provided with a first (24) and a second end (24'), generally opposite each other,
- an anchoring element (13) having a proximal end (51) and a distal end (52), wherein the proximal end (51) is arranged at the first end (24) of the connecting element (20) and a fastening means (18) is arranged at the second end (24') of the connecting element (20), and
- a clamping means (74) which connects the connecting element (20) to the fastening element (18),
**characterized in that** the fastening means (18) has the following:
- a tubular element (25);
- a connecting element (27) of the fastening means (18), wherein the connecting element (27) has a bracket (28) with a free end (30), which is arranged pivotably in a tubular element (25) of the fastening means (18);
and
- a gripping element (60), wherein
- the connecting element (27) of the fastening means (18) has a free end (30) which is arranged pivotably in the tubular element (25), and
- a leg spring (38) is pivotably arranged at the other end (54) of the pivotable connecting element (27) of the fastening means (18), which end is opposite the longitudinal axis (33) in the longitudinal direction (53), on which leg spring, again firmly connected thereto, two spring arms (50, 50') are arranged spaced apart in parallel by the leg spring (38).

2. The heart valve implant (11) according to claim 1, **characterized in that** the tubular element (25) forms a joint-like connection (29) with the connecting element (27) of the fastening means (18) and the connecting element (27) of the fastening means (18) forms a joint-like connection (29') with the gripping element (60).

3. The heart valve implant (11) according to claim 1 to 2, **characterized in that** the tubular element (25) is formed as a cylindrical sleeve (25') and has two openings (31, 31') opposite each other in the sleeve wall (32), lying on a transverse axis (35), wherein the transverse axis (35) is perpendicular to the longitudinal axis (33) of the sleeve (25') and the openings (31, 31'), viewed in the longitudinal direction of the sleeve (25'), are arranged centrally in the sleeve wall (32).

4. The heart valve implant (11) according to claim 1, **characterized in that** the connecting element (27) of the fastening means (18) forms a bracket (28) comprising two longitudinal legs (34, 34') and a transverse leg (36), wherein a pin or an eyelet are arranged at the free ends (30) of the longitudinal legs (34, 34') and the transverse leg (36) forms a connecting rod (37) with a transverse axis (44) which is spaced parallel to the transverse axis (35) in the sleeve (25') and, as connecting rod (37), is the carrier of a leg spring (38).

5. The heart valve implant (11) according to claim 1, **characterized in that** the leg spring (38) of the gripping element (60) is formed from a leaf spring (39) in a U-shape and has at least two leaf spring legs (45, 45', 49, 49'), wherein a connecting element (41) with a transverse axis (55) corresponding to the connecting rod (37) of the bracket (28) is arranged in the inner apex (48) of the leg region (40).

6. The heart valve implant (11) according to claim 5, **characterized in that** the connecting element (41) is formed from at least an annular eyelet (42, 42'), a cylindrical channel (43), a longitudinal groove (43) or a slit (72).

7. The heart valve implant (11) according to claim 5 to 6, **characterized in that** the connecting element (41) is arranged on the outside (70) of the leaf spring legs (45, 45', 49, 49') in the leg region (48) of the side facing away from the opening (47) of the leaf spring legs (45, 45', 49, 49').

8. The heart valve implant (11) according to claim 5, **characterized in that** the leaf spring (39) of the leg spring (38) has at least one recess (71) in the surface (70) in the leaf spring leg (45, 49).

9. The heart valve implant (11) according to claim 1, **characterized in that** the gripping element (60) has a jaw part (56) formed by the spring arms (50, 50') which is arranged on the fastening side (63) of the gripping element (60) and on the side facing away from the opened leg spring (38) and resting on the longitudinal axis (33) of the tubular element (25), wherein the jaw part (56) has at least one spacer (88) arranged in the jaw part (56), which creates a predetermined gap (89) between the toothing (87) of the gripping jaws (86) in the jaw part (56).

10. A heart valve implant system for the minimally invasive repair of a valve flap (6) in the beating heart (1) of a patient, with
- an outer tubular pusher (57) with lumen for guiding and holding a fastening means (18);
- a first inner tubular pusher I (58) with lumen for opening and closing a gripping element (60);
- a second inner tubular pusher II (59) with lumen for guiding and screwing in an anchoring element (13);
- a third inner tubular pusher III (77) for inserting and positioning a sliding ring (76); and
- a heart valve implant (11), having:
- a connecting element (20), wherein the connecting element (20) is provided with a first (24) and a second end (24'), generally opposite each other;
- an anchoring element (13) having a proximal end (51) and a distal end (52), wherein the proximal end (51) is arranged at the first end (24) of the connecting element (20) and a fastening means (18) is arranged at the second end (24') of the connecting element (20);
- a fastening means (18) which has a tubular element (25);
- a clamping means (74) which connects the connecting element (20) to the fastening element (18); and
- a connecting element (27) of the fastening means (18);
wherein
- the connecting element (27) of the fastening means (18) has a bracket (28) which has a free end (30) which is arranged pivotably in the tubular element (25),
- a gripping element (18) is pivotably arranged at the other end (54) of the pivotable connecting element (27) of the fastening means (18), which end is opposite the longitudinal axis (33) in the longitudinal direction (53), and
- the gripping element (60) has a leg spring (38) on which, firmly connected thereto, two spring arms (50, 50') are arranged spaced apart in parallel by the leg spring (38).

11. The heart valve implant system according to claim 10, **characterized in that** the gripping element (60) has a jaw part (56) formed by the spring arms (50, 50') which is arranged on the fastening side (63) of the gripping element (60) and on the side facing away from the opened leg spring (38) and resting on the longitudinal axis (33) of the tubular element (25), wherein the jaw part (56) has at least one spacer (88) arranged in the jaw part (56), which spacer creates a predetermined gap (89) between the toothing (87) of the gripping jaws (86) in the jaw part (56).

## Revendications

1. Implant valvulaire cardiaque (11) destiné à la réparation mini-invasive d'une valvule (6) dans le coeur battant (1) d'un patient, comportant
- un élément de liaison (20), l'élément de liaison (20) étant pourvu d'une première (24) et d'une seconde extrémité (24'), généralement opposées l'une à l'autre,
- un élément d'ancrage (13) présentant une extrémité proximale (51) et une extrémité distale (52), l'extrémité proximale (51) étant disposée à la première extrémité (24) de l'élément de liaison (20) et un moyen de fixation (18) étant disposé à la seconde extrémité (24') de l'élément de liaison (20), et
- un moyen de serrage (74) reliant l'élément de liaison (20) à l'élément de fixation (18),
**caraetérisé** en ce que ledit moyen de fixation (18) présente les éléments suivants :
- un élément tubulaire (25) ;
- un élément de liaison (27) du moyen de fixation (18), l'élément de liaison (27) présentant un étrier (28) pourvu d'une extrémité libre (30) qui est logée de manière à pouvoir pivoter dans un élément tubulaire (25) du moyen de fixation (18) ;
et
- un élément de préhension (60), dans lequel
- l'élément de liaison (27) du moyen de fixation (18) présentant une extrémité libre (30) logée de manière à pouvoir pivoter dans l'élément tubulaire (25), et
- l'autre extrémité (54) de l'élément de liaison (27) pivotant du moyen de fixation (18), opposée dans le sens longitudinal (53) à l'axe longitudinal (33), comportant un ressort à branches (38) disposé de manière à pouvoir pivoter, muni de deux bras à ressort (50, 50') espacés parallèlement par le ressort à branches (38) et à leur tour solidaires de celui-ci.

2. Implant valvulaire cardiaque (11) selon la revendication 1, **caraetérisé** en ce que l'élément tubulaire (25) forme une liaison articulée (29) avec l'élément de liaison (27) du moyen de fixation (18) et que l'élément de liaison (27) du moyen de fixation (18) forme une liaison articulée (29') avec l'élément de préhension (60).

3. Implant valvulaire cardiaque (11) selon les revendications 1 à 2, **caractérisé en ce que** l'élément tubulaire (25) est réalisé sous la forme d'un manchon (25') cylindrique et présente deux ouvertures (31, 31') opposées, ménagées dans la paroi du manchon (32), se trouvant sur un axe transversal (35), l'axe transversal (35) étant perpendiculaire à l'axe longitudinal (33) du manchon (25') et les ouvertures (31, 31') étant disposées, vues dans le sens longitudinal du manchon (25'), au milieu de la paroi du manchon (32).

4. Implant valvulaire cardiaque (11) selon la revendication 1, **caractérisé en ce que** l'élément de liaison (27) du moyen de fixation (18) forme un étrier (28) qui comprend deux branches longitudinales (34, 34') et une branche transversale (36), un tenon ou un oeillet étant disposés aux extrémités libres (30) des branches longitudinales (34, 34') et la branche transversale (36) formant une barre de liaison (37) à axe transversal (44), qui est espacée parallèlement à l'axe transversal (35) dans le manchon (25') et qui est porteuse d'un ressort à branches (38) en tant que barre de liaison (37).

5. Implant valvulaire cardiaque (11) selon la revendication 1, **caractérisé en ce que** le ressort à branches (38) de l'élément de préhension (60) est réalisé en forme de U à partir d'un ressort à lames (39) et présente au moins deux branches du ressort à lames (45, 45', 49, 49'), un élément de liaison (41) correspondant à la barre de liaison (37) de l'étrier (28) et présentant un axe transversal (55) étant disposé au niveau du sommet (48) de la zone des branches (40), côté intérieur.

6. Implant valvulaire cardiaque (11) selon la revendication 5, **caractérisé en ce que** l'élément de liaison (41) est composé d'au moins un oeillet annulaire (42, 42'), d'un canal cylindrique (43), d'une rainure longitudinale (43) ou d'une fente (72).

7. Implant valvulaire cardiaque (11) selon les revendications 5 à 6, **caractérisé en ce que** l'élément de liaison (41) est disposé sur le côté extérieur (70) des branches du ressort à lames (45, 45', 49, 49') dans la zone des branches (48) du côté opposé à celui de l'ouverture (47) des branches du ressort à lames (45, 45', 49, 49').

8. Implant valvulaire cardiaque (11) selon la revendication 5, **caractérisé en ce que** le ressort à lames (39) du ressort à branches (38) présente au moins un évidement (71) dans la branche du ressort à lames (45, 49) à la surface (70).

9. Implant valvulaire cardiaque (11) selon la revendication 1, **caractérisé en ce que** l'élément de préhension (60) présente un mors (56) formé par les bras à ressort (50, 50'), positionné à plat du côté de la fixation (63) de l'élément de préhension (60) et sur le côté opposé du ressort à branches (38) ouvert ainsi que sur l'axe longitudinal (33) de l'élément tubulaire (25), le mors (56) disposant d'au moins un écarteur (88) placé dans le mors (56), qui crée une fente (89) prédéterminée entre la denture (87) des mâchoires de préhension (86) dans le mors (56).

10. Système d'implant valvulaire cardiaque destiné à la réparation mini-invasive d'une valvule (6) dans le coeur battant (1) d'un patient, comportant
- une coulisse tubulaire extérieure (57) à lumière pour guider et maintenir un moyen de fixation (18) ;
- une première coulisse tubulaire intérieure I (58) à lumière pour ouvrir et fermer un élément de préhension (60) ;
- une deuxième coulisse tubulaire intérieure II (59) à lumière pour guider et visser un élément d'ancrage (13) ;
- une troisième coulisse tubulaire intérieure III (77) pour insérer et positionner une bague coulissante (76) ; et
- un implant valvulaire cardiaque (11), présentant :
- un élément de liaison (20), l'élément de liaison (20) étant pourvu d'une première (24) et d'une seconde extrémité (24'), généralement opposées l'une à l'autre ;
- un élément d'ancrage (13) présentant une extrémité proximale (51) et une extrémité distale (52), l'extrémité proximale (51) étant disposée à la première extrémité (24) de l'élément de liaison (20) et un moyen de fixation (18) étant disposé à la seconde extrémité (24') de l'élément de liaison (20) ;
- un moyen de fixation (18) présentant un élément tubulaire (25) ;
- un moyen de serrage (74) reliant l'élément de liaison (20) à l'élément de fixation (18) ; et
- un élément de liaison (27) du moyen de fixation (18) ;
- l'élément de liaison (27) du moyen de fixation (18) présentant un étrier (28) pourvu d'une extrémité libre (30) qui est logée de manière à pouvoir pivoter dans l'élément tubulaire (25),
- l'autre extrémité (54) de l'élément de liaison (27) pivotant du moyen de fixation (18), opposée dans le sens longitudinal (53) à l'axe longitudinal (33), comportant un élément de préhension (60) disposé de manière à pouvoir pivoter, et
- l'élément de préhension (60) présentant un ressort à branches (38) muni de deux bras à ressort (50, 50') espacés parallèlement par le ressort à branches (38) et solidaires de celui-ci.

11. Système d'implant valvulaire cardiaque selon la revendication 10, **caractérisé en ce que** l'élément de préhension (60) présente un mors (56) formé par les bras à ressort (50, 50'), positionné à plat du côté de la fixation (63) de l'élément de préhension (60) et sur le côté opposé du ressort à branches (38) ouvert ainsi que sur l'axe longitudinal (33) de l'élément tubulaire (25), le mors (56) disposant d'au moins un écarteur (88) disposé dans le mors (56), qui crée une fente (89) prédéterminée entre la denture (87) des mâchoires de préhension (86) dans le mors (56).
